(19) Europäisches Patentamt
European Patent Office
Office européen des brevets

(11) **EP 4 076 181 B1**

(12) **EUROPEAN PATENT SPECIFICATION**

(45) Date of publication and mention
of the grant of the patent:
**23.07.2025 Bulletin 2025/30**

(21) Application number: **20838933.8**

(22) Date of filing: **16.12.2020**

(51) International Patent Classification (IPC):
*A61B 5/11* (2006.01)    *A61B 5/107* (2006.01)
*A61B 5/00* (2006.01)    *A61B 5/01* (2006.01)
*A61F 13/00* (2024.01)

(52) Cooperative Patent Classification (CPC):
**A61B 5/1121; A61B 5/01; A61B 5/1071;
A61B 5/6831; A61B 5/6833;** A61B 2560/0252;
A61B 2560/0412; A61B 2562/0261; A61B 2562/164;
A61B 2562/187

(86) International application number:
**PCT/EP2020/086361**

(87) International publication number:
**WO 2021/122701 (24.06.2021 Gazette 2021/25)**

(54) **MONITORING AND THERAPY DEVICES**

ÜBERWACHUNGS- UND THERAPIEVORRICHTUNGEN

DISPOSITIFS DE SURVEILLANCE ET DE THÉRAPIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.12.2019 GB 201918475
18.12.2019 GB 201918680**

(43) Date of publication of application:
**26.10.2022 Bulletin 2022/43**

(73) Proprietor: **T.J.Smith and Nephew,Limited
Hull
HU3 2BN (GB)**

(72) Inventors:
• **HUNT, Allan, Kenneth, Frazer, Grugeon**
Hull HU3 2BN (GB)
• **KOTECHA, Bindiya**
Hull HU3 2BN (GB)
• **PARTINGTON, Lee, Ian**
Hull HU3 2BN (GB)
• **STRACHAN, Kirsty, Margaret**
Hull HU3 2BN (GB)
• **URWIN, Charlotte, Rose**
Hull HU3 2BN (GB)
• **WARREN-BARRATT, Emily, Grace**
Retford Nottinghamshire DN22 7JP (GB)

(74) Representative: **Appleyard Lees IP LLP
15 Clare Road
Halifax HX1 2HY (GB)**

(56) References cited:
EP-A1- 3 466 323      WO-A1-2017/195038
WO-A1-2018/093275     WO-A1-2018/187027
WO-A1-2019/042790     WO-A1-2019/048638
WO-A1-2019/096828     WO-A2-2019/020550
WO-A2-2019/063488     US-A1- 2018 271 409
US-B2- 10 274 305

• VOTZKE CALLEN ET AL: "Highly-Stretchable
Biomechanical Strain Sensor using Printed
Liquid Metal Paste", 2018 IEEE BIOMEDICAL
CIRCUITS AND SYSTEMS CONFERENCE
(BIOCAS), IEEE, 17 October 2018 (2018-10-17),
pages 1 - 4, XP033483141, DOI: 10.1109/
BIOCAS.2018.8584671

- NAKAMOTO HIROYUKI ET AL: "Stretchable Strain Sensor With Anisotropy and Application for Joint Angle Measurement", IEEE SENSORS JOURNAL, IEEE SERVICE CENTER, NEW YORK, NY, US, vol. 16, no. 10, 1 May 2016 (2016-05-01), pages 3572 - 3579, XP011605901, ISSN: 1530-437X, [retrieved on 20160408], DOI: 10.1109/JSEN.2016.2535489
- MORTEZA AMJADI ET AL: "Ultra-stretchable and skin-mountable strain sensors using carbon nanotubes-Ecoflex nanocompos", NANOTECHNOLOGY, INSTITUTE OF PHYSICS PUBLISHING, GB, vol. 26, no. 37, 25 August 2015 (2015-08-25), pages 375501, XP020289460, ISSN: 0957-4484, [retrieved on 20150825], DOI: 10.1088/ 0957-4484/26/37/375501
- TAIRYCH ANDREAS ET AL: "Distributed sensing: multiple capacitive stretch sensors on a single channel", PROCEEDINGS OF SPIE; [PROCEEDINGS OF SPIE ISSN 0277-786X VOLUME 10524], SPIE, US, vol. 10163, 17 April 2017 (2017-04-17), pages 1016306 - 1016306, XP060090183, ISBN: 978-1-5106-1533-5, DOI: 10.1117/12.2260416

## Description

The present application claims priority from GB Patent Application No. 1918475.3, filed on December 16, 2019, titled RANGE OF MOTION MONITORING APPARATUS and GB Patent Application No. 1918680.8, filed on December 18, 2019, titled SYSTEMS AND METHODS FOR MONITORING MOTION, RANGE OF MOTION, AND SHAPE OF A BODY PART.

Field

Some arrangements of the present disclosure relate to apparatuses, systems, and methods for the measuring of range of movement and angle of bend of joints and/or for the monitoring of motion, range of motion, and shape of a body part, which can be applicable to monitoring or treatment of pressure ulcers.

Description of the Related Art

Contemporary devices for determining range of movement (ROM) are often very basic and inconvenient, requiring an actual doctor's visit or visit to the physiotherapist, or can be highly sophisticated and expensive. Because of the limitations in available apparatuses for determine ROM, determination of the progress of healing of a patient, for example, can be greatly delayed.
Pressure ulcers, which are also known as pressure sores, bedsores, or decubitus ulcers, are injuries to skin and underlying tissue resulting from prolonged pressure on the skin, soft tissue, muscle, or bone above capillary filling pressure (approximately 32 mmHg). One type of pressure ulcer that develops on a foot is known as a diabetic foot ulcer (DFU), which tends to occur with higher frequency and intensity in the diabetic population. Management and treatment of diabetic foot ulcers requires offloading the wound by using cushioned footwear, such as a support boot, cast, shoe, etc. While offloading can be effective, because many offloading devices are removable, it has been found that patient non-compliance with the offloading devices plays a large role in the delayed healing of diabetic foot ulcers.
However, prior art approaches and systems provide no or little information regarding patients' lifestyle and non-compliance or compliance with the offloading devices and support surfaces. Gaining insight into patients' lifestyle can be important for prevention and healing of pressure ulcers. However, because of these limitations, prevention and healing of patients' pressure ulcers using prior art approaches and systems may be delayed or, worse yet, the condition could worsen leading to prolonged discomfort, hospitalization, or even surgery.
WO2019/096828 relates to a wound monitoring and/or therapy system which includes a substantially stretchable substrate supporting a plurality of electronic components.
WO2019/042790 relates to systems, devices and methods for monitoring dimensional changes in medical devices attached to or implanted in the body, such as wound fillers. VOTZKE CALLEN ET AL: "Highly-Stretchable Biomechanical Strain Sensor using Printed Liquid Metal Paste",2018 IEEE BIOMEDICAL CIRCUITS AND SYSTEMS CONFERENCE (BIOCAS), IEEE, 17 October 2018 (2018-10-17), pages 1-4, XP033483141,DOI: 10.1109/ BIOCAS.2018.8584671, discloses a strain sensor.

SUMMARY OF THE INVENTION

The invention is defined by an apparatus according to claim **1**.
Further embodiments are defined by dependent claims **2-15**.

**SUMMARY OF SOME EXEMPLIFYING ARRANGEMENTS**

A monitoring and/or therapy apparatus includes a contact layer having a stretchable or substantially stretchable substrate configured to be attached to a user's joint. The apparatus includes at least one strain sensor coupled with the contact layer. The apparatus includes electronic circuitry electronically coupled with the at least one strain sensor. The electronic circuitry is configured to determine an amount of an elongation of the at least one strain sensor based on a change in a resistance of the at least one strain sensor. The at least one strain sensor includes a plurality of stretchable tracks positioned on the substrate. The resistance of the at least one strain sensor increases as the at least one strain sensor is elongated due to the contact layer being elongated. The electronic circuitry is configured to determine an angle and/or a range of movement of the joint responsive to changes in the resistance of the at least one strain sensor.
The apparatus of any of the preceding or foregoing paragraphs and/or any of the apparatuses disclosed herein can include one or more of the following features. The plurality of stretchable tracks can include conductive ink printed on the substrate. The electronic circuitry includes a machine-readable memory storing executable instructions and being configured to record information related to an elongation of the at least one strain sensor. The electronic circuitry can be, but is not required to be, coupled with the contact layer. The electronic circuitry can be spaced apart from the contact layer. The electronic circuitry can be positioned at least partially within a housing that can be configured to be positionable at a distance from the contact layer so that the electronic circuitry can be spaced apart from the contact layer, or positioned within a housing that can be coupled with or in contact with the contact layer.
The apparatus of any of the preceding or foregoing paragraphs and/or any of the apparatuses disclosed herein can include one or more of the following features. The at least one strain sensor and/or the electronic circuitry can be positioned on or can be printed on an edge portion of the contact layer, or the at least one strain sensor and/or the electronic circuitry can be positioned on or can be printed outside of a middle portion of the contact layer so that the at least one strain sensor and the electronic circuitry can be positioned on a peripheral portion of the contact layer. The at least one strain sensor and/or the electronic circuitry can be at least partially covered by or encapsulated in at least one coating or protective film.

The apparatus of any of the preceding or foregoing paragraphs and/or any of the apparatuses disclosed herein can include one or more of the following features. The apparatus can include a wireless communications device electronically coupled with the electronic circuitry. The wireless communications device can be configured to transmit a signal containing an angle and/or a range of movement of the joint. The wireless communications device can be configured to transmit a signal containing at least one of an angle or a range of movement of the joint responsive to changes in the resistance of the at least one strain sensor. The apparatus can include a Bluetooth communications device electronically coupled with the electronic circuitry, a source of power for at least the electronic circuitry, wherein the source of power can be coupled with the contact layer, and/or one or more flexible batteries.

The apparatus of any of the preceding or foregoing paragraphs and/or any of the apparatuses disclosed herein can include one or more of the following features. The at least one strain sensor can include a first strain sensor and a second strain sensor spaced apart from the first strain sensor. The at least one strain sensor can, but is not required to, include a first strain sensor and a second strain sensor, wherein the second strain sensor can be parallel to the first strain sensor. The at least one strain sensor can include three strain sensors parallel and spaced apart from one another.

The apparatus of any of the preceding or foregoing paragraphs and/or any of the apparatuses disclosed herein can include one or more of the following features. The electronic circuitry can be configured to automatically collect data from the strain sensor at a predetermined frequency. The electronic circuitry can be configured to automatically gather readings from the strain sensor at a plurality of time intervals, and shorten a time interval in response to the strain sensor experiencing changes in the resistance at a higher frequency. The electronic circuitry can be configured to increase a frequency of data collected from the strain sensor in response to the strain sensor experiencing at least one of a higher frequency or a greater magnitude of changes in the resistance.

The apparatus of any of the preceding or foregoing paragraphs and/or any of the apparatuses disclosed herein can include one or more of the following features. The apparatus can include a temperature sensor. The temperature sensor can be configured to provide to the electronic circuitry an electrical signal related to at least one of a temperature of the strain sensor or a temperature of a region of the substrate adjacent to the strain sensor. The temperature sensor can be positioned on the substrate adjacent to the strain sensor, or can be integrated with the strain sensor. The temperature sensor can be positioned on the substrate adjacent to the strain sensor or portion of the strain sensor, wherein the temperature sensor can be configured to provide an electrical signal indicative of a temperature of the strain sensor and the electronic circuitry can be further configured to calibrate readings from the strain sensor based on the temperature of the strain sensor. The apparatus can include an alarm, and the electronic circuitry can be further configured to activate the alarm in response to a resistance of the strain sensor satisfying a threshold resistance.

The apparatus of any of the preceding or foregoing paragraphs and/or any of the apparatuses disclosed herein can include one or more of the following features. The contact layer can be formed from a substantially impermeable material, or can be configured to provide a substantially impermeable cover over a joint on the body. The contact layer can be configured to have a shape which substantially matches a joint when the contact layer is in a relaxed state, or to have a generally curved shape when the contact layer is in a relaxed state, or have a tubular shape. The contact layer can be configured to have a shape which substantially matches a joint when the contact layer can be in a relaxed state, wherein the joint can be an elbow joint, a wrist joint, a knee joint, a neck joint, a spine, a finger joint, or an ankle joint. The apparatus can be configured to work with any joint on the body (human, animal, or otherwise), wherein the joint can be a wrist, elbow, knee, ankle, neck, spine, shoulder joint, or other joint of the body.

The apparatus of any of the preceding or foregoing paragraphs and/or any of the apparatuses disclosed herein can include one or more of the following features. The contact layer can be formed from a flexible material, and can include adhesive. In some arrangements, the contact layer can include a first adhesive segment attached to a first surface of the contact layer, a second adhesive segment attached to the first surface of the contact layer, and a space between the first and second adhesive segments not including any adhesive.

The apparatus of any of the preceding or foregoing paragraphs and/or any of the apparatuses disclosed herein can include one or more of the following features. The contact layer can be configured to be positioned over a wound. The apparatus can include a source of negative pressure in fluid communication with a space under the contact layer, the source of negative pressure being configured to aspirate fluid from a wound over which the contact layer can be positioned. The apparatus can include a port in fluid communication with the contact layer configured to communicate a source of reduced pressure to a wound over which the contact layer can be positioned.

A range of movement sensor apparatus (which can be similar to the apparatuses described in the preceding paragraphs and/or any of the apparatuses described herein) can include a sensor having a flexible substrate sized and configured to be attached to a skin surface of a user adjacent to a joint. The apparatus can include a strain sensor having a resistance strain gauge coupled with the substrate. The strain gauge can be configured to provide data related to an amount of an elongation and/or a curvature of a region of the substrate adjacent to the strain sensor to a processor. The range of movement sensor apparatus can include a machine-readable memory storing executable instructions, and a processor in communication with the machine-readable memory and the strain sensor. The processor can be configured to execute the instructions stored on the machine-readable memory to cause the processor to automatically collect one or more readings from the strain sensor.

An apparatus having a strain sensor (sometimes referred to herein as a stretchable sensor), which can be a capacitive strain sensor (also referred to herein as a capacitive strain gauge or a capacitive load cell), can be made using an electroactive polymer. The apparatus may be used to measure at least one of the motion, range of motion, shape or stiffness of joints or body parts of a human, animal, or other living user. The apparatus can be configured to infer or calculate an offloading of body parts from the measurements taken from the strain sensor. In any arrangements, the strain sensor can include a dielectric material or layer (which can, but is not required to, comprise an electroactive polymer) between two electrodes. The strain sensor can be encased in a silicone isolator. An adhesive (such as, but not limited to, a silicone adhesive) can be used between the strain sensor and/or a contact layer or other material, substrate, or object that the strain sensor is coupled with and the body surface to ensure intimate contact under strain when the tissues move beneath the strain sensor or the contact layer, or otherwise.

Any arrangements of the apparatuses disclosed herein can be used for monitoring and/or therapy. Further. the apparatus of any of the preceding paragraphs and/or any of the apparatuses disclosed herein can include one or more of the following features. The apparatus can have a contact layer configured to be attached to a tissue of a user, a strain sensor coupled with the contact layer, and electronic circuitry electronically coupled with the strain sensor. The electronic circuitry can be configured to determine a level of at least one of an elongation or a curvature of the strain sensor based on changes in the capacitance of the strain sensor. Further, the electronic circuitry can be configured to determine that the contact layer is subjected to at least one of elongation or curvature that satisfies a threshold and, optionally, provide an indication that the contact layer is subjected to the at least one of elongation or curvature that satisfies the threshold. In any arrangements disclosed herein, the strain sensor can be configured to provide data to the electronic circuitry related to an amount of the curvature of the contact layer adjacent to the strain sensor. For example and without limitation, the strain sensor of any arrangements disclosed herein can be a strain sensor (sometimes referred to herein as a strain gauge) configured such that, for example, a capacitance of the strain sensor changes responsive to the strain sensor being elongated and/or curved. In any arrangements disclosed herein, the strain sensor can have an electroactive polymer positioned between a first and a second electrode. In any embodiments disclosed herein, a first portion of the strain sensor can be coupled with the contact layer, a second portion of the strain sensor can be coupled with the contact layer, and a space between the first portion of the strain sensor and the second portion of the strain sensor can be configured to not be coupled with the contact layer such that a space can exist between the first and second portions of the strain sensor wherein the strain sensor is not coupled with the contact layer. Further, in any arrangements disclosed herein, any portion of the strain sensor, including without limitation the first and second portions of the strain sensor, can be welded, ultrasonic welded, and/or thermally bonded to the contact layer, or bonded to or coupled with the contact layer using any suitable materials and/or methods.

The strain sensor can be integrated with the contact layer, or the strain sensor can have adhesive on at least a portion of a surface of the contact layer. The contact layer can have a first adhesive segment attached to a first surface of the strain sensor, a second adhesive segment attached to the first surface of the strain sensor, and a space between the first and second adhesive segments that does not include or have any adhesive. The second adhesive segment can have a different stiffness as compared to the first adhesive segment, or can have the same stiffness or substantially the same stiffness as compared to the first adhesive segment. In any arrangements disclosed herein, the contact layer can be formed from a substantially impermeable material.

The apparatus of any of the preceding or foregoing paragraphs and/or any of the apparatuses disclosed herein can include one or more of the following features. The apparatus can be configured such that the electronic circuitry determines if the contact layer is experiencing elongation and/or if the contact layer is experiencing curvature. In any arrangements disclosed herein, the electronic circuitry can be coupled with the contact layer. The electronic circuitry can have machine-readable memory. The electronic circuitry and/or the machine-readable memory can be coupled with the contact layer. In any arrangements disclosed herein, the apparatus can have a source of power for at least the electronic circuitry, wherein the source of power can have one or more rechargeable batteries, one or more flexible batteries, or other sources of power. In any arrangements disclosed herein, the at least one strain sensor and/or any portion of, or all of, the electronic circuitry can be positioned on or can be printed on an edge portion of the contact layer.

The apparatus of any of the preceding or foregoing paragraphs and/or any of the apparatuses disclosed herein can include one or more of the following features. In any arrangements disclosed herein, the strain sensor can have a first strain gauge (or sensor) and a second strain gauge (or sensor), wherein a capacitance of the first capacitive strain gauge changes responsive to the first strain gauge being at least one of elongated and curved, and a capacitance of the second strain gauge changes responsive to the second strain gauge being at least one of elongated and curved. In any arrangements, the strain sensor can have a first strain gauge and a second strain gauge, wherein the first strain gauge can be positioned above the second strain gauge and can be coupled with the second strain gauge in alignment with the first strain gauge. The first and second strain gauges (which can be capacitive strain gauges) can be identical in any arrangements, or the first strain gauge can have a different size, thickness, and/or shape as compared to the second strain gauge. In some arrangements, the first and second strain gauges can be arranged in a sandwiched configuration, for example a club sandwich type configuration. For example and without limitation, in some arrangements, the first capacitive strain gauge can have a first electrode and a second electrode and the second capacitive strain gauge can have a first electrode. The second electrode of the first capacitive strain gauge can be a second electrode for the second capacitive strain gauge.

The electronic circuitry of any arrangements disclosed herein can be configured to calculate a difference between an elongation of the first strain gauge and an elongation of the second strain gauge to determine an amount of at least one of an elongation or curvature of the contact layer. Any arrangements of the strain sensor disclosed herein can have a spacer layer between the first strain gauge and the second strain gauge, wherein the spacer layer can be configured to increase a difference between an elongation of the first strain gauge and an elongation of the second strain gauge. The strain sensor can have a non-continuous spacer layer between the first strain gauge and the second strain gauge, wherein the spacer layer can be configured to decrease a stiffness of the strain sensor. The apparatus of any arrangements disclosed herein can have a non-continuous spacer layer between the first strain gauge and the second strain gauge, optionally, wherein the non-continuous spacer layer can be configured to cause the strain sensor to perform as multiple discrete strain sensors rather than a single continuous strain sensor.

In some arrangements, the spacer layer can include a plurality of layers sandwiched together, or can include a plurality of materials. In some arrangements, the strain sensor can have a stiffening layer between the first strain gauge (which can be a first capacitive strain gauge) and the second strain gauge (which can be a first capacitive strain gauge). The stiffening layer can be configured to inhibit or attenuate deformations from the first strain gauge from affecting the second strain gauge and/or to inhibit or attenuate deformations from the second strain gauge from affecting the first strain gauge. In some arrangements, the strain sensor can have a cushioning layer between the first capacitive strain gauge and the second capacitive strain gauge. The stiffening layer can be configured to inhibit or attenuate deformations from the first capacitive strain gauge from affecting the second capacitive strain gauge and/or to inhibit or attenuate deformations from the second capacitive strain gauge from affecting the first capacitive strain gauge.

The apparatus of any of the preceding or foregoing paragraphs and/or any of the apparatuses disclosed herein can include one or more of the following features. In any arrangements disclosed herein, the at least one strain sensor can have a first strain sensor and a second strain sensor spaced apart from the first strain sensor (wherein any of the strain sensors is a single strain sensor or a stacked strain sensor). In some arrangements, the at least one strain sensor can include a first strain sensor at a first position and a second strain sensor at a second position, wherein the first position and the first strain sensor at least partially overlaps the second position and the second strain sensor. Further, in any arrangements, the at least one strain sensor can include a first strain sensor at a first orientation and a second strain sensor at a second orientation, wherein the first orientation is different than the second orientation.

The second strain sensor can be parallel to the first strain sensor, in line with the first strain sensor, spaced apart and at an oblique angle relative to the first strain sensor, or otherwise. For example and without limitation, an apparatus can have two, three, four, or more, or from two to five, or from three to four, strain sensors in series, in parallel, or in any desired arrangement (including, without limitation, any of the arrangements disclosed above).

The apparatus of any of the preceding or foregoing paragraphs and/or any of the apparatuses disclosed herein can include one or more of the following features. The electronic circuitry and/or the machine-readable memory can be within a housing that is separate from the contact layer, coupled with the contact layer, integrally formed with the contact layer, or otherwise. In any arrangements disclosed herein, the electronic circuitry can be positioned at least partially within a housing that is configured to be at least one of positionable at a distance from the contact layer so that the electronic circuitry can be spaced apart from the contact layer, or coupled with the contact layer. In any arrangements disclosed herein, the electronic circuitry can have a machine-readable memory storing executable instructions and being configured to record information related to an elongation of the at least one strain sensor.

The apparatus of any of the preceding or foregoing paragraphs and/or any of the apparatuses disclosed herein can include one or more of the following features. In any arrangements disclosed herein, the apparatus can have a wireless communications device electronically coupled with the electronic circuitry, the wireless communications device configured to transmit a signal associated with at least one of an elongation or a curvature of the strain sensor.

The apparatus of any of the preceding or foregoing paragraphs and/or any of the apparatuses disclosed herein can include one or more of the following features. In any arrangements disclosed herein, the electronic circuitry can be configured to automatically collect data from the strain sensor at a predetermined frequency. The electronic circuitry can be configured to automatically gather readings from the strain sensor at least one predetermined time interval. The apparatus can be configured such that the at least one time interval decreases when the strain sensor experiences changes in strain at a higher frequency so that the electronic circuitry gathers data from the strain sensor at a higher frequency when the strain sensor experiences changes in strain at a higher frequency. In any arrangements disclosed herein, the electronic circuitry can be configured to automatically gather readings from the strain sensor at a plurality of time intervals, and shorten a time interval in response to the strain sensor experiencing changes in the capacitance at a higher frequency. The electronic circuitry can be configured to increase a frequency of data collected from the strain sensor when the strain sensor experiences a higher frequency of changes and/or a greater magnitude of changes in the elongation and/or curvature.

The apparatus of any of the preceding or foregoing paragraphs and/or any of the apparatuses disclosed herein can include one or more of the following features. In any arrangements disclosed herein, the apparatus can have a temperature sensor in electrical communication with the electronic circuitry. The temperature sensor can be configured to provide an electrical signal indicative of a temperature of the strain sensor and the electronic circuitry can be configured to calibrate the readings from the strain sensor based on the temperature of the strain sensor. The temperature sensor can be configured to enable the electronic circuitry to determine a temperature of the strain sensor or of the contact layer adjacent to the strain sensor. The temperature sensor can be adjacent to the strain sensor. In any arrangements, the temperature sensor can be configured to provide to the electronic circuitry an electrical signal related to at least one of a temperature of the strain sensor or a temperature of a region of the substrate adjacent to the strain sensor. Any arrangements disclosed herein can that can have a temperature sensor positioned on the substrate adjacent to the strain sensor or portion of the strain sensor, and wherein the temperature sensor can be configured to provide an electrical signal indicative of a temperature of the strain sensor and the electronic circuitry can be configured to calibrate readings from the strain sensor based on the temperature of the strain sensor. The temperature sensor can be adjacent to the strain sensor or part of the strain sensor.

The apparatus of any of the preceding or foregoing paragraphs and/or any of the apparatuses disclosed herein can include one or more of the following features. The apparatus of any of the preceding claims, wherein the electronic circuitry can be configured to activate an indicator when a threshold level of elongation and/or a threshold level of curvature of the contact layer can be detected by the electronic circuitry and the strain sensor.

In any arrangements disclosed herein, the apparatus can have an alarm and can be configured to activate the alarm when a threshold level of curvature of the contact layer can be detected by the electronic circuitry and the strain sensor.

The apparatus of any of the preceding paragraphs and/or any of the apparatuses disclosed herein can include one or more of the following features. In any arrangements disclosed herein, the contact layer can be configured to have a shape which substantially matches a joint when the contact layer can be in a relaxed state. In any arrangements disclosed herein, the contact layer can be configured to have a shape which substantially matches a joint when the contact layer can be in a relaxed state, and wherein the joint can be an elbow joint, a wrist joint, a knee joint, a neck joint, a spine, a finger joint, an ankle joint, a shoulder joint, a hip joint, or a toe joint. In any arrangements, the contact layer can have a tubular shape.

The apparatus of any of the preceding or foregoing paragraphs and/or any of the apparatuses disclosed herein can include one or more of the following features. In any arrangements disclosed herein, the contact layer can be configured to be positioned over a wound, and the apparatus can have a source of negative pressure in fluid communication with a space under the contact layer. The source of negative pressure can be configured to aspirate fluid from a wound over which the contact layer can be positioned, but is not required to. Any arrangements of the apparatuses and/or methods disclosed herein can be configured to provide negative pressure to the space between the contact layer and the wound, with or without aspiration. Any arrangements disclosed herein can have a port in fluid communication with the contact layer configured to communicate a source of reduced pressure to a space between the contact layer and a wound.

**In** any arrangements, the contact layer can include a laminate of one or more different materials. Further, in any arrangements of the apparatuses disclosed herein, the contact layer can have an adhesive on at least one surface thereof.

Disclosed herein are arrangements of a monitoring and/or therapy apparatus that can have a contact layer configured to be attached to a tissue of a user, one or more temperature sensors coupled with a tissue of a user or the contact layer, and one or more strain sensors coupled with or adjacent to the one or more temperature sensors. In some arrangements, the one or more strain sensors can be configured to measure a strain of the contact layer and/or a tissue of the user in response to the one or more strain sensors being at least one of elongated and curved, and electronic circuitry electronically coupled with the one or more temperature sensors and the one or more strain sensors. The electronic circuitry can be configured to determine a level of at least one of an elongation or a curvature of the one or more strain sensors. Further, in any arrangements, the electronic circuitry can be configured to determine if the strain of the contact layer and/or the tissue is greater than a threshold value to determine if the one or more temperature sensors are adequately coupled with the tissue of the user or the contact layer. In some arrangements, the electronic circuitry can be configured to determine a temperature of the tissue of the user or of the contact layer with the one or more temperature sensors. Further, some arrangements of the apparatuses disclosed herein can include a plurality of strain sensors in an array with the one or more temperature sensors.

The apparatus of any of the preceding or foregoing paragraphs and/or any of the apparatuses disclosed herein can include one or more of the following features. A negative pressure wound therapy apparatus, having a wound cover that can have an impermeable contact layer sized and configured to be attached to a skin surface of a user and to cover a wound, and a strain sensor configured to determine a strain by passing an electrical signal through the strain sensor. The apparatus of any arrangements disclosed herein can have a machine-readable memory having stored thereon executable instructions, and a processor in communication with the machine-readable memory and the strain sensor. The strain sensor can be coupled with the contact layer and can be configured such that the capacitance of the strain sensor changes as the strain sensor is elongated or curved. The processor can be configured to execute the instructions stored on the machine-readable memory to cause the processor to automatically determine the capacitance of the strain sensor at a predetermined frequency and, from the capacitance of the strain sensor, to determine a level of an amount of an elongation and/or a curvature of the contact layer adjacent to the strain sensor. The strain sensor can be configured to provide data to the processor related to an amount of the curvature of the contact layer adjacent to the strain sensor.

The apparatus of any of the preceding or foregoing paragraphs and/or any of the apparatuses disclosed herein can include one or more of the following features. A negative pressure wound therapy apparatus having a wound cover that can have an impermeable contact layer sized and configured to be attached to a skin surface of a user and to cover a wound, and a strain sensor that can have a first strain sensor and a second strain sensor coupled with the contact layer. The strain sensor can be configured to provide data related to an amount of an elongation and/or a curvature of the contact layer adjacent to the strain sensor to a processor. Any arrangements can have a machine-readable memory having stored thereon executable instructions, and a processor in communication with the machine-readable memory and the strain sensor, wherein the processor can be configured to execute the instructions stored on the machine-readable memory to cause the apparatus to automatically gather one or more readings from the strain sensor. The first strain sensor can be configured to provide data associated with an elongation of the contact layer associated with a stretching or a bending of the contact layer adjacent to the first strain sensor to the processor, and the second strain sensor can be configured to provide data associated with an elongation of the contact layer associated with a stretching or a bending of the contact layer adjacent to the second strain sensor to the processor.

Some arrangements disclosed herein relate to a method (no methods by themselves form part of the claims) of operating and/or using the apparatus of any of the preceding paragraphs and/or described herein is disclosed.

## BRIEF DESCRIPTION OF THE DRAWINGS

Other features and advantages of the present disclosure will be apparent from the following detailed description, taken in conjunction with the accompanying drawings of which:

Figure 1A is a side view of a user's leg having a sensor apparatus for a user's knee and a sensor apparatus for a user's ankle.
Figure 1B is a front view of the sensor apparatus for the user's knee shown in Figure 1A.
Figure 1C is a front view of the sensor apparatus for the user's ankle shown in Figure 1A.
Figure 1D is a back view of the sensor apparatus for the user's ankle shown in Figure 1A.
Figure 2 illustrates a monitoring and/or therapy apparatus.
Figures 3 to 8 illustrate monitoring and/or therapy apparatuses.
Figure 9 illustrates a side view of an arrangement of a strain sensor that can be used with any of the monitoring and/or therapy apparatus arrangements disclosed herein.
Figure 10 illustrates a side view of another arrangement of a strain sensor that can be used with any of the monitoring and/or therapy apparatus arrangements disclosed herein.
Figure 11 illustrates a side view of another arrangement of a strain sensor that can be used with any of the monitoring and/or therapy apparatus arrangements disclosed herein.

Figure 12 illustrates the arrangement of the strain sensor of Figure 3 transitioning from a flat state or shape to a curved state or shape.

Figure 13 illustrates a strain sensor in a bent state.

Figure 14 illustrates another arrangement of a strain sensor.

## DETAILED DESCRIPTION

Some systems and methods (no methods by themselves form part of the claims) disclosed herein relate to wound therapy for a human or animal body. Any reference to a wound herein can refer to a wound on a human or animal body, and any reference to a body herein can refer to a human or animal body. The disclosed technology arrangements may relate to preventing or minimizing damage to physiological tissue or living tissue, or to the treatment of damaged tissue (for example, a wound as described herein) wound with or without reduced pressure, including for example a source of negative pressure and wound dressing components and apparatuses. The apparatuses and components comprising the wound overlay and packing materials or internal layers, if any, are sometimes collectively referred to herein as dressings. In some cases, the wound dressing can be provided to be utilized without reduced pressure.

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno-compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, pressure injury, or epidermolysis bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, burns, diabetic ulcers, pressure ulcers, pressure injury, stoma, surgical wounds, trauma and venous ulcers or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

Wounds may also include a pressure injury. A pressure injury is localized damage to the skin and/or underlying soft tissue, usually over a bony prominence or related to a medical or other device. The injury can present as intact skin or an open ulcer and may be painful. The injury occurs as a result of intense and/or prolonged pressure or pressure in combination with shear. The tolerance of soft tissue for pressure and shear may also be affected by microclimate, nutrition, perfusion, comorbidities and condition of the soft tissue.

Wound may also include tissue at risk of becoming a wound as discussed herein. For example, tissue at risk may include tissue over a bony protuberance (at risk of deep tissue injury/insult) or pre-surgical tissue (for example, knee tissue) that may have the potential to be cut (for example, for joint replacement/surgical alteration/reconstruction).

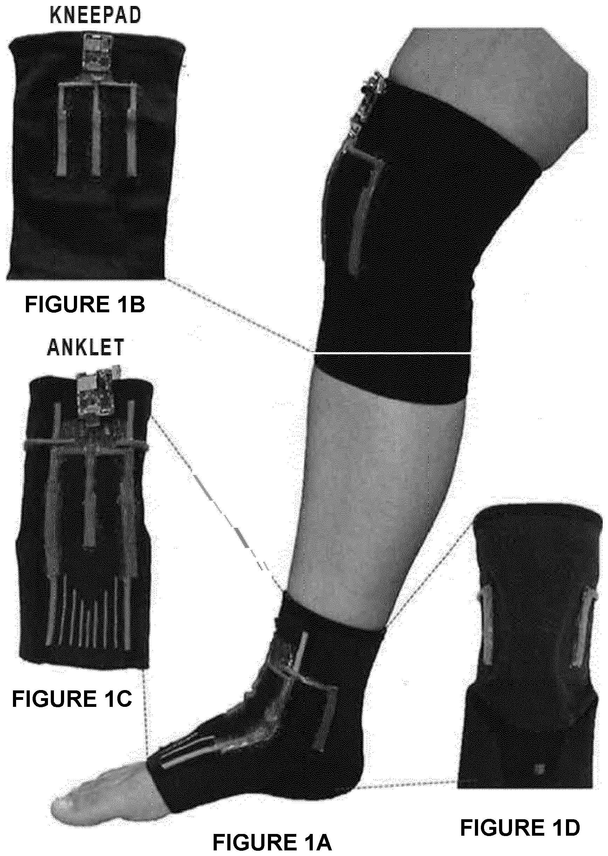

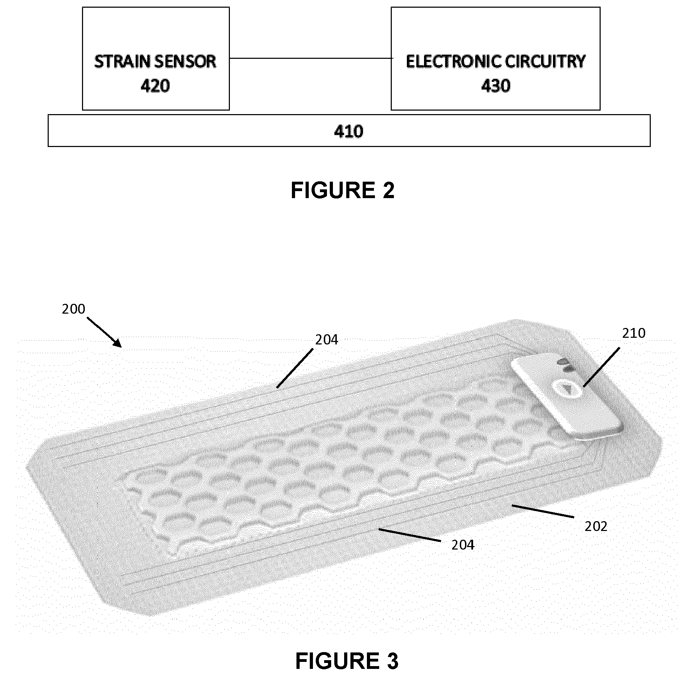

With reference to Figures 1A - 1D and 2, any arrangements of the monitoring and/or therapy apparatuses disclosed herein can include a contact layer (for example, illustrated as 410 in Figure 2), at least one strain sensor (for example, illustrated as 420 in Figure 2) coupled with the contact layer, and electronic circuitry (for example, illustrated as 430 in Figure 2) electronically coupled with the at least one strain sensor. In some arrangements, the contact layer can have, for example and without limitation, a stretchable or substantially stretchable substrate such as, without limitation, polyurethane, thermopolyurethane (TPU), silicone, polycarbonate, polyethylene, polyimide, polyamide, polyester, polyethelene tetraphthalate (PET), polybutalene tetreaphthalate (PBT), polyethylene naphthalate (PEN), polyetherimide (PEI), along with various fluropolymers (FEP) and copolymers, a Smith and Nephew polyurethane extensible film, Allevyn dressing manufactured by Smith & Nephew, Rensys dressing manufactured by Smith & Nephew. IV3000 dressing manufactured by Smith & Nephew, PICO RETENTION STRIPS manufactured by Smith & Nephew, or another suitable material

In some arrangements, the electronic circuitry can be configured to determine an amount of an elongation of the at least one strain sensor based on a change in a resistance of the at least one strain sensor. The electronic circuitry can include one or more processors or controllers, memories, or the like. The at least one strain sensor can have a plurality of stretchable tracks positioned on the substrate, and can be configured such that the resistance of the at least one strain sensor increases as the at least one strain sensor is elongated due to the contact layer being elongated.

The apparatus can determine elongation of the cover layer by measuring specific changes in the conductive track resistance of the strain sensor, which increases as the material is stretched. By measuring changes in resistance of the tracks when the substrate is stretched around the joint of a limb, the extent of the stretch can be used to deduce the angle, and hence the range of movement (ROM) of the joint. In some cases, ROM can refer to range of motion.

In some arrangements, the apparatus and/or the substrate can be configured to be attached to or adjacent to a user's joint. In any arrangements disclosed herein, the plurality of stretchable tracks can have or be made from a conductive material, such as copper applied to the substrate, conductive ink printed on the substrate, conductive glue applied to the substrate or the like. For example and without limitation, the plurality of stretchable tracks can be made from or include a silver ink, carbon ink, graphite ink, or other type of conductive ink.

Some arrangements of the apparatus can be configured to measure a range of movement, and can have a sensor device, a flexible substrate sized and configured to be attached to a skin surface of a user adjacent to a joint, and a strain sensor that can have a resistance strain gauge coupled with the substrate. The apparatus can further have a machine-readable memory having stored thereon executable instructions, and a processor in communication with the machine-readable memory and the strain sensor. The strain gauge can be configured to provide data related to an amount of an elongation and/or a curvature of a region of the substrate adjacent to the strain sensor to a processor. In some arrangements, the processor can be configured to execute the instructions stored on the machine-readable memory to cause the processor to automatically collect one or more readings from the strain sensor. In any arrangements, the apparatus can collect data without computing range of movement (ROM).

Some arrangements of the monitoring and/or therapy apparatus disclosed herein comprise stretchable electronics (that can include a strain gauge, or a plurality of strain gauges) that can be integrated along or coupled with a contact layer. For example, with reference to Figure 3, monitoring and/or therapy apparatus 200 can include a contact layer 202, one or more (two being shown) strain gauges 204 extending along a length of the contact layer, and electronic circuitry 210 electronically coupled with the strain gauges 204. The strain gauges 204 can be positioned on the contact layer 202 so as to extend along a length of an adhesive border of the contact layer so that the strain gauges 204 are spaced apart from a middle portion of the contact layer (which can be positioned over a wound) for electrical safety. In other arrangements, the strain gauges 204 and/or electronic circuitry can be positioned within any layer up to and including the top film of the contact layer (which can also be referred to or be a dressing in any arrangements disclosed herein).

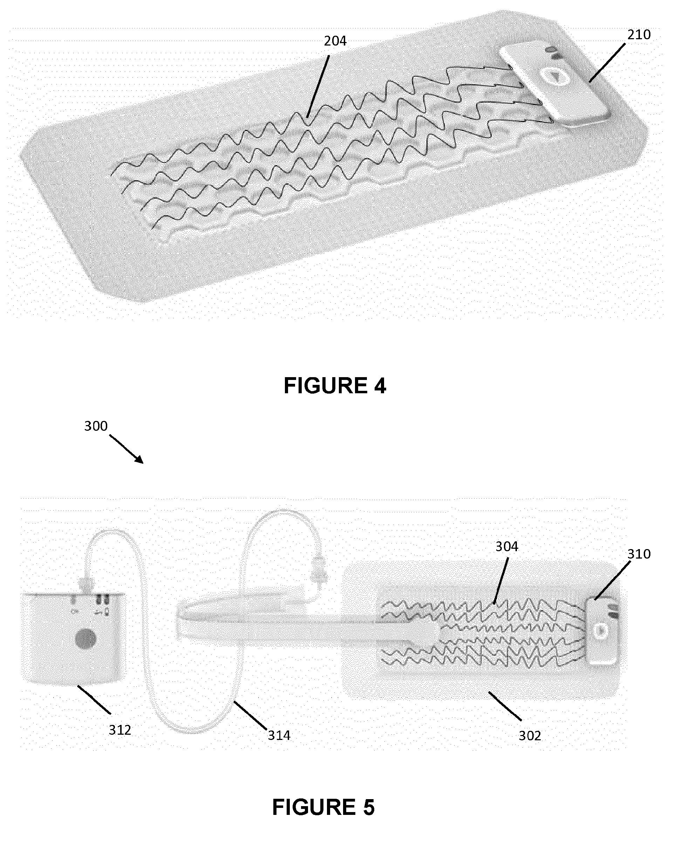

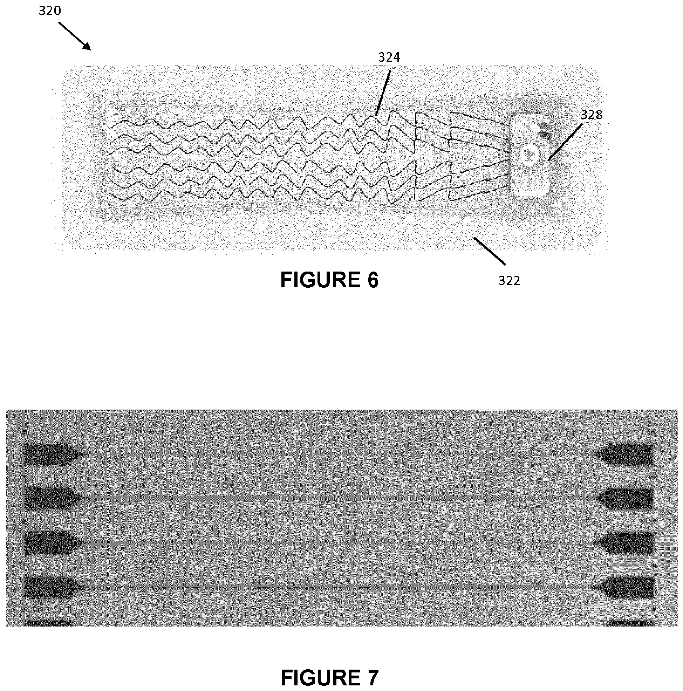

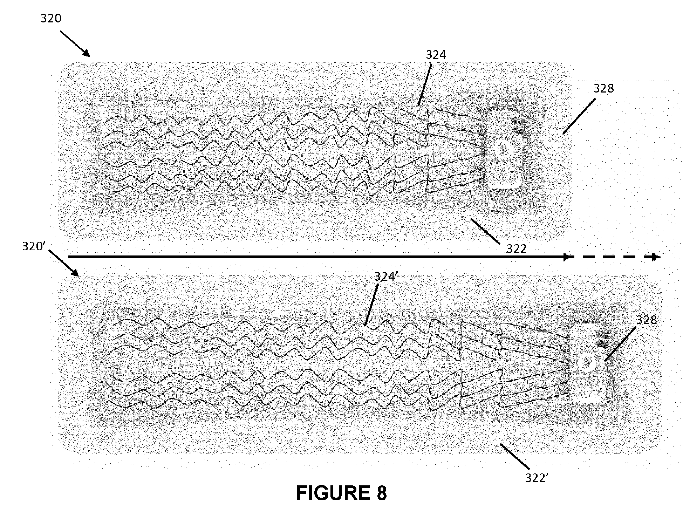

Figure 5 illustrates an arrangement of a monitoring and/or therapy apparatus 300 that comprises a wound dressing 302, extensible tracks 304 of a strain sensor, a housing 310 comprising electronic circuitry, and a portable pump apparatus 312 in fluid communication with the dressing 302 using a conduit or tube 314. Any arrangements of the strain sensors disclosed herein can be used in place of the strain sensor shown in Figure 5. Figure 6 illustrates another arrangement of a monitoring and/or therapy apparatus 320 comprising a dressing 322 (that can be an ALLEVYN dressing, as shown), extensible tracks 324 of a strain sensor, and a housing 328 comprising electronic circuitry. As shown, the extensible tracks 324 follow a wavy pattern and cover a substantial portion of a surface of the dressing, and extend substantially the entire length of the dressing. Figure 8 illustrates the arrangement of the apparatus 320 shown in Figure 6 in a first or relaxed state and in a second or elongated state, wherein the entire dressing and the conductive tracks are elongated. In particular, the apparatus 320 in the relaxed state is shown in an upper portion of Figure 8. The apparatus 320' in the elongated state may be the results of a bending of a joint or other elongation of the skin or tissue under the dressing or contact layer 322'. As the dressing or contact layer 322' is elongated, such as when, for example, the skin or tissue beneath the contact layer 322' elongates, the conductive tracks 324' are also configured to elongate. The strain gauge can be used to measure or calculate the extent of elongation of the dressing or contact layer.

In some arrangements, when a dressing or contact layer is stretched, for example, over a knee joint after knee replacement surgery, the dressing will extend from a baseline state to a strained or elongated state, which will change the resistance reading. The change can be linked to the level of stretch, to calibrate, a strong verification testing package to train the architecture on what resistance values equate to in degrees extension/-contraction of a particular joint. For example, a change in X resistance on a knee can correspond to Y degrees movement. In real use, in some arrangements, this may require some initial set up, either on board the control box with cyclic settings selection e.g. elbow, knee, ankle etc. where the software could record the baseline resistances in the relaxed position or state coupled with clear instructions on where to apply in each case. Or, in other arrangements, an app could record information such as patient biometrics (height etc.), position of dressing (potentially via photo) to calibrate or normalize the data observed over time.

In any arrangements disclosed herein, the strain sensor can have any number of conductive tracks, including six tracks (as shown in Figure 6), or one or more, or from two to ten or more, or from four to eight conductive tracks. With reference to Figure 7, the conductive tracks of any apparatus or strain gauge arrangement disclosed herein can have a generally straight shape or path.

Additionally, if the stretchable electronics (which comprises the strain gauge or gauges) are positioned in a fluid pathway of a wound dressing, such as a negative pressure wound therapy dressing, the stretchable electronics can be encapsulated in a protective material. For example and without limitation, individual films with printed electronic tracks could be placed along the length of the dressing (like bed slats), or one continuous film that is appropriately perforated or slit to permit fluid transfer to the absorbent inner materials of the dressing.

In any arrangements, the electronic circuitry (such as, but not limited to, the electronic circuitry 210 shown in Figure 3) can comprise a small, simple control box housing power, memory and communication components. The electronic circuitry can be spaced apart from the contact layer, or can be integrated into the top film of a contact layer or dressing for an all in one solution. The power supply could be from a number of sources including flexible batteries to aid conformability.

In any arrangements disclosed herein, the contact layer (which can be a wound dressing) can be extensible so as to stretch with the tissue under and/or adjacent to the contact layer. The apparatus can be integrated into any desired dressing shape, format or type, including any of the dressings shown in the attached figures or described herein. For example and without limitation, a compatible dressing to integrate any arrangements of the apparatuses and/or strain sensors disclosed herein can include an ALLEVYN dressing, which can be configured to have greater extensibility for application over knee and elbow joints. Additionally, any arrangements of the apparatuses and/or strain sensors disclosed herein can be used with contact layers or dressings comprising thermoplastic polyurethane, or polyurethane extensible film, in addition to, for example and without limitation, RENASYS DRAPE dressings, IV3000 dressings, PICO RETENTION STRIPS, etc.

For any arrangements, the resistance gauges comprising conductive tracks could be positioned on or at a top film level away from the skin, or encapsulated between a plurality of at least two layers of film, or encapsulated with conformal coatings (for example, as described herein) for electrical safety and waterproof properties so that, for example, a patient or user can shower without needing to remove the apparatus.

The track designs of the strain gauges of any arrangements disclosed herein can be any desired design, including straight, oscillating waveforms in various frequencies and amplitudes, crosslinked, zig-zag, etc. Nonlimiting examples of patterns of track designs of the strain gauges 204 are shown in Figures 3 - 8. In any arrangements disclosed herein, the tracks can be implemented or positioned in the cover layer or dressing border, dressing body, or both.

Determination of ROM and Angle

Range of movement (ROM) is defined as the rotation about a joint and is an essential measurement across a number of clinical practices including orthopaedic, musculoskeletal, and neurological. This measure is particularly useful in patients who have undergone joint replacement, or those who have suffered from broken bones where range of movement becomes limited during the healing process and requires physio therapy when the fracture is healed. Target joints may be any, though not limited to the following: wrist, elbow, knee, ankle, neck, spine, and/or shoulder.

In any arrangements disclosed herein, the electronic circuitry can be configured to determine an angle and/or a range of movement of the joint responsive to changes in the resistance of the at least one strain sensor.

Current methods of determining ROM can require a face to face appointment with a doctor or physio to take the range of movement measurement with equipment as simple as a protractor, or can require complex and expensive wearables or visioning systems such as Optitrack or Vicon 3D. The arrangements of the apparatuses disclosed herein can provide lower cost and much more convenient alternatives to contemporary ROM devices.

Electronic Circuitry

In any arrangements, a small, simple control box housing power, memory and communication components, and other components of the electronic circuitry, could be integrated into the top film of the dressing for an all in one solution. The power supply could be from a number of sources including flexible batteries to aid conformability.

In any arrangements, the electronic circuitry can be coupled with the contact layer or otherwise positioned on or carried by the contact layer. For example and without limitation, at least some of or at least a portion of the electronic circuitry can be printed directly on the contact layer, or can be supported by or partially or completely enclosed within a housing that is coupled with the contact layer. Alternatively, the electronic circuitry can be positioned at least partially within a housing that can be configured to be positionable at a distance from the contact layer so that the electronic circuitry can be spaced apart from the contact layer, or carried by or at a different part of the body.

In any arrangements disclosed herein, the electronic circuitry that can have a machine-readable memory storing executable instructions and being configured to record information related to an elongation of the at least one strain sensor.

In any arrangements disclosed herein, the at least one strain sensor and/or the electronic circuitry can be positioned on or printed directly on an edge portion of the contact layer, or can be printed directly on or positioned outside of a middle portion of the contact layer so that the at least one strain sensor and the electronic circuitry can be positioned on a peripheral portion of the contact layer.

In some arrangements, the electronics (which can be, but are not required to be, stretchable) could be integrated along the length of the adhesive border so that the electronics are clearly distanced from the wound for electrical safety or otherwise, or within any layer up to and including the top film. In any arrangements, the electronics can be encapsulated in a protective material. Individual films with printed electronic tracks could be placed along the length of the dressing, or one continuous film that is appropriately perforated or slit to permit fluid transfer to the absorbent inner materials of the dressing.

Communications Devices

Any of the arrangements of the apparatus disclosed herein can have a wired or wireless communications device electronically coupled with the electronic circuitry. The wireless communications device can be configured to transmit a signal containing an angle and/or a range of movement of the joint. Further, in any arrangements, the wireless communications device can be configured to transmit a signal containing at least one of an angle or a range of movement of the joint responsive to changes in the resistance of the at least one strain sensor. In some arrangements, the apparatus can have a bluetooth communications device electronically coupled with the electronic circuitry.

The wired or wireless communications device can be configured to transmit information to a separate computing device, such as a computer (for example, clinician's computer), server, mobile computing device, pager, monitoring unit hospital monitoring system, or the like. In any arrangements, the information recorded or saved by the apparatus, and/or transmitted by the apparatus, can include strain sensor data (including maximum, minimum, and average values over a duration of time), range of movement information (including maximum, minimum, and average values over a duration of time), joint angle information (including maximum, minimum, and average values over a duration of time), usage data, and/or alarm conditions,.

The communications device can transmit the information via one or more of Near Field Communication (NFC), Bluetooth™, Bluetooth™ Low Energy, Zigbee, radio waves, Wi-Fi, or the like. The communications device can, for example and in some arrangements, communicate and pair with other devices (such as, without limitation, a smart phone, a tablet computer, or a desktop computer) and transmit the information in an encrypted or protected format. Additionally, in any arrangements disclosed herein, the apparatus and/or the communications device can also be configured to receive signals or information including, for example and without limitation, operating instructions for the device and/or data from an outside source. For example, the device can be configured to receive a signal that can cause the apparatus to trigger an alarm on the device, cause the device to enter a state of hibernation or lower power, or cause the device to change a frequency of data collection, or some other operating parameter of the device.

Source of Power

In any arrangements disclosed herein, the apparatus can have a source of power for at least the electronic circuitry. The source of power can be located separate from the electronics circuitry, or coupled with the electronics circuitry, or otherwise. In some arrangements, the source of power can be coupled with or supported by the contact layer. In any arrangements disclosed herein, the source of power can have one or more flexible batteries and can be positioned on the contact layer. The source of power can be rechargeable (such as a lithium ion battery) or replaceable, or can be permanently coupled with the apparatus so as to not be replaceable.

Multiple Sensors

In any arrangements disclosed herein, the at least one strain sensor can include a first strain sensor and a second strain sensor spaced apart from the first strain sensor. The first and the second strain sensors can have a plurality of stretchable tracks positioned on the substrate. The first and the second strain sensors can be configured such that a resistance of the first and the second strain sensors increases as the first and second strain sensors are elongated due to the contact layer being elongated. Further, the electronic circuitry can be configured to determine at least one of an angle or a range of movement of the joint responsive to changes in the resistance of the first and the second strain sensors.

In any arrangements disclosed herein, the at least one strain sensor can include a first strain sensor and a second strain sensor, wherein the second strain sensor can be positioned so as to be parallel to the first strain sensor. In any arrangements disclosed herein, wherein the at least one strain sensor can include three strain sensors parallel and spaced apart from one another.

Frequency of Data Collection

In any arrangements disclosed herein, the electronic circuitry is further configured to automatically collect data from the strain sensor at a predetermined frequency or (not according to the invention) over a predetermined length of time at a predetermined or varying frequency, or can be further configured to automatically gather readings from the strain sensor at a plurality of time intervals, and shorten a time interval in response to the strain sensor experiencing changes in the resistance at a higher frequency. The electronic circuitry can be further configured to increase a frequency of data collected from the strain sensor in response to the strain sensor experiencing at least one of a higher frequency or a greater magnitude of changes in the resistance. In one application, the apparatus can be used to evaluate patient compliance with exercise or ROM movements, or to identify patients not engaging in physiotherapy/stretch exercises.

In some arrangements, the apparatus can be configured to report daily values, such as max or min values, average values, or the like. Such information can be used to provide feedback to a user to inform the user regarding the progress of treatment, or regression of his/her/its condition.

Temperature Sensor

Any arrangements of the apparatus disclosed herein further has a temperature sensor, wherein the temperature sensor is configured to provide to the electronic circuitry an electrical signal related to a temperature of the strain sensor and/or a temperature of a region of the substrate adjacent to the strain sensor. The temperature sensor can be positioned on the substrate adjacent to the strain sensor. In some arrangements, the temperature sensor can be integrated with the strain sensor. In any arrangements disclosed herein, the temperature sensor can be positioned on the substrate adjacent to the strain sensor or portion of the strain sensor, and can be configured to provide an electrical signal indicative of a temperature of the strain sensor. The electronic circuitry can be further configured to calibrate readings from the strain sensor based on the temperature of the strain sensor.

Alarm

In any arrangements disclosed herein, the apparatus can further have an alarm, and wherein the electronic circuitry can be further configured to activate the alarm in response to a resistance of the strain sensor satisfying a threshold resistance so as to indicate a threshold elongation of the cover layer, or a threshold value of range of movement, and/or a threshold bend angle of the joint.

Contact Layer Details

In any arrangements disclosed herein, the contact layer can be formed from a substantially impermeable material and/or be configured to provide a substantially impermeable cover over the joint. In any arrangements, the contact layer can be formed from a flexible material and/or can have an adhesive. For example and without limitation, the contact layer can have a first adhesive segment attached to a first surface of the substrate or contact layer, a second adhesive segment attached to the first surface of the substrate or contact layer, and a space between the first and second adhesive segments not including any adhesive. The contact layer can be configured to conform to the body's geometry without impeding natural movement.

Additionally, in any arrangements disclosed herein, the contact layer can be configured to have a shape which substantially matches a joint when the contact layer is in a relaxed state, wherein the joint can be an elbow joint, a wrist joint, a knee joint, a neck joint, an ankle joint, or any other joint on the body of a human, animal, or other living creature, such as a horse, a dog, or otherwise. In any arrangements, the contact layer can have a curved shape when the contact layer is in a relaxed state, or can have a tubular shape.

Joint

Further, the apparatus of any of the arrangements disclosed herein can be configured for use on any desired body part, including without limitation a wrist, elbow, knee, ankle, neck, spine, and/or shoulder joint. For example and without limitation, the contact layer can be configured to have a shape which substantially matches a joint when the contact layer is in a relaxed state, wherein the joint can be, without limitation, an elbow joint, a wrist joint, a knee joint, a neck joint, or an ankle joint. Additionally, the contact layer can be configured to have a shape which substantially matches a plurality of different joints when the contact layer is in a relaxed state, so that the contact layer can be more universally applied, wherein the joint can be, without limitation, an elbow joint, a wrist joint, a knee joint, a neck joint, a spine, a finger joint, and or an ankle joint.

Wound Dressing

In any arrangements disclosed herein, wherein the contact layer can be configured to be positioned over a wound. The apparatus can have a source of negative pressure in fluid communication with a space under the contact layer, the source of negative pressure being configured to aspirate fluid from a wound over which the contact layer is positioned. The apparatus can further have a port in fluid communication with the contact layer and configured to communicate a source of reduced pressure to a wound over which the contact layer is positioned.

For example and without limitation, any arrangements of the apparatus disclosed herein can be integrated into or used with an Allevyn or Allevyn I POST OP wound dressing manufactured by Smith & Nephew. The strain sensor technology could be integrated into one of several layers within an Allevyn dressing or other postoperative dressing. When the dressing is applied over the affected joint, extension or flexion of the limb causes the tracks to be stretched, increasing their resistance relative to the resting position which may indicate the angle of movement occurring.

In some arrangements, the sensor technology described herein can be integrated into an adhesive film substrate without absorbent capabilities for applications when this may not be required, for example, where there is no external wound e.g. broken bone, ligament/tendon/-soft tissue damage. This film could have a high breathability to reduce maceration on the application area.

Encapsulation and Stress Relief

In some cases, while it may be desirable for a substrate to be stretchable or substantially stretchable to better conform to or cover the joint or wound, at least some of the electronic circuitry, strain sensor, or any other electronic components or connections may not be stretchable or flexible. In such instances, undesirable or excessive localized strain or stress may be exerted on the one or more electronic components or connections, such as on the supporting area or mountings of an electronic component or connection, when the substrate is positioned in or over the joint or wound. For example, such stress can be due to patient movement, changes in the shape or size of the joint or wound (such as, due to its healing), or the like. Such stress may cause movement, dislodgment, or malfunction of the one or more electronic components or connections (for example, creation of an open circuit from a pin or another connector becoming disconnected). Alternatively or additionally, it may be desirable to maintain the position of one or more electronic components or connections in the same or substantially same location or region with respect to the joint or wound (such as, in contact with the joint or wound) so that measurements collected by the one or more electronic components accurately capture changes over time in the same or substantially same location or region of the joint or wound. While the surface of the stretchable substrate may move when, for example, the patient moves, it may be desirable to maintain same or substantially same locations of one or more electronic components or connections relative to the joint or wound.

To address these problems, in some cases, non-stretchable or substantially non-stretchable coating (such coating can sometimes be referred to as "hard coat") can be applied to one or more electronic components, one or more electronic connections, or the like. Hard coat can provide one or more of reinforcement or stress relief for one or more electronic components, one or more electronic connections, or the like. Hard coating can be formed from acrylated or modified urethane material. For example, hard coat can be one or more of Dymax 1901-M, Dymax 9001-E, Dymax 20351, Dymax 20558, Henkel Loctite 3211, or another suitable material. Hard coat can have viscosity from about 13,500cP to 50,000cP before being cured or from about 3,600cP to about 6,600cP before being cured. In some cases, hard coat can have viscosity of no more than about 50,000cP. Hard coat can have hardness from about D40 to about D65 and/or linear shrinkage of about 1.5-2.5%.

In some cases, another coating (or coatings) can be applied to encapsulate or coat one or more of the substrate or components supported by the substrate, such as the electronic connections or the electronic components. Coating can provide biocompatibility, shield or protect the electronics from coming into contact with fluids, provide padding for the electronic components to increase patient comfort, or the like. As used herein, biocompatible can mean being in compliance with one or more applicable standards, such as ISO 10993 or USP Class VI. Such coating can be sometimes referred to as "conformal coat" or "soft coat." Soft coat can be stretchable or substantially stretchable. Soft coat can be hydrophobic or substantially hydrophobic.

Soft coat can be formed from one or more suitable polymers, adhesives, such as 1072-M adhesive (for example, Dymax 1072-M), 1165-M adhesive (such as, Dymax 1165-M), parylene (such as, Parylene C), silicones, epoxies, urethanes, acrylated urethanes, acrylated urethane alternatives (such as, Henkel Loctite 3381), or other suitable biocompatible and substantially stretchable materials. Soft coat can be thin coating, for example, from about 80 microns or less up to several millimeters or more. Soft coat can have hardness lower than about A100, A80, A50 or lower. Soft coat can have elongation at break higher than about 100%, 200%, 300% or more. Soft coat can have viscosity of about 8,000-14,500 centipoise (cP). In some cases, coating can have viscosity no less than about 3,000cP. In some cases, coating can have viscosity less than about 3,000cP.

Any of the hard or soft coats described herein can be applied by one or more of laminating, adhering, welding (for instance, ultrasonic welding), curing by one or more of light, UV, thermal (such as, heat), or the like. Any of the hard or soft coat described herein can be transparent or substantially transparent to facilitate optical sensing. Any of the coatings described herein can retain bond strength when subjected to sterilization, such as EtO sterilization. Any of the coatings described herein can be modified to fluoresce, such as under UV light.

Other Variations

Although this disclosure describes certain arrangements, it will be understood by those skilled in the art that many aspects of the methods and devices shown and described in the present disclosure may be differently combined and/or modified to form still further arrangements or acceptable examples. All such modifications and variations are intended to be included herein within the scope of this disclosure. Indeed, a wide variety of designs and approaches are possible and are within the scope of this disclosure. No feature, structure, or step disclosed herein is essential or indispensable. Moreover, while illustrative arrangements have been described herein, the scope of any and all arrangements having equivalent elements, modifications, omissions, combinations (for example, of aspects across various arrangements), substitutions, adaptations and/or alterations as would be appreciated by those in the art based on the present disclosure. While certain arrangements have been described, these arrangements have been presented by way of example only, and are not intended to limit the scope of protection.

Features, materials, characteristics, or groups described in conjunction with a particular aspect, arrangement, or example are to be understood to be applicable to any other aspect, arrangement or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

While certain arrangements have been described, these arrangements have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some arrangements, the actual steps taken in the processes illustrated and/or disclosed may differ from those shown in the figures. Depending on the arrangement, certain of the steps described above may be removed, others may be added. For example, the actual steps and/or order of steps taken in the disclosed processes may differ from those shown in the figure. Depending on the arrangement, certain of the steps described above may be removed, others may be added. For instance, the various components illustrated in the figures may be implemented as software and/or firmware on a processor, controller, ASIC, FPGA, and/or dedicated hardware. Hardware components, such as processors, ASICs, FPGAs, and the like, can include logic circuitry.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some arrangements, the actual steps taken in the processes illustrated and/or disclosed may differ from those shown in the figures. Depending on the arrangement, certain of the steps described above may be removed, others may be added. Furthermore, the features and attributes of the specific arrangements disclosed above may be combined in different ways to form additional arrangements, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular arrangement. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain arrangements include, while other arrangements do not include, certain features, elements, and/or steps. Thus, such conditional language is not generally intended to imply that features, elements, and/or steps are in any way required for one or more arrangements or that one or more arrangements necessarily include logic for deciding, with or without user input or prompting, whether these features, elements, and/or steps are included or are to be performed in any particular arrangement. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain arrangements require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain arrangements, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

The language of the claims is not limited to the examples described in the present specification or during the prosecution of the application, which examples are to be construed as non-exclusive.

Additional arrangements disclosed can be used for the monitoring of loading of a body part, position of a body part, motion of a body part, range of movement of a body part, or the like. Any of the disclosed arrangements can be used for monitoring and/or treatment of pressure ulcers. Loading can refer to transferring or placing at least a threshold amount of force on a body part. Placing such threshold amount of force on the body part causes the body part to support weight. For example, loading of a foot can refer to transferring or placing at least a portion of the body weight (or body weight in combination with external weight) on the foot such that the foot is supporting at least such portion of the body weight. At least such portion of the body weight can serve as a threshold for determining that the foot has been loaded.

Some of the disclosed implementations can be utilized with a dressing (also sometimes referred to as wound dressing) alone or in combination with negative or reduced pressure. The apparatuses and components including an overlay and packing materials or internal layers, if any, are sometimes collectively referred to herein as dressings.

Some arrangements disclosed herein relate to wound monitoring or therapy for a human or animal body. Therefore, any reference to a wound herein can refer to a wound on a human or animal body, and any reference to a body herein can refer to a human or animal body. The disclosed technology arrangements may relate to preventing or minimizing damage to physiological tissue or living tissue, or to the treatment of damaged tissue (for example, a wound as described herein).

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno-compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, or epidermolysis bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, bums, diabetic ulcers, pressure ulcers, stoma, surgical wounds, trauma and venous ulcers or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

Wounds may also include a pressure injury. A pressure injury is localized damage to the skin and/or underlying soft tissue, usually over a bony prominence or related to a medical or other device. The injury can present as intact skin or an open ulcer and may be painful. The injury occurs as a result of intense and/or prolonged pressure or pressure in combination with shear. The tolerance of soft tissue for pressure and shear may also be affected by microclimate, nutrition, perfusion, comorbidities and condition of the soft tissue.

Wound may also include tissue at risk of becoming a wound as discussed herein. For example, tissue at risk may include tissue over a bony protuberance (at risk of deep tissue injury/insult) or pre-surgical tissue (for example, knee tissue) that may have the potential to be cut (for example, for joint replacement/surgical alteration/reconstruction).

Some arrangements relate to methods of monitoring or treating a wound with the technology disclosed herein in conjunction with one or more of the following: advanced footwear, turning a patient, offloading (such as, offloading diabetic foot ulcers), treatment of infection, systemix, antimicrobial, antibiotics, surgery, removal of tissue, affecting blood flow, physiotherapy, exercise, bathing, nutrition, hydration, nerve stimulation, ultrasound, electrostimulation, oxygen therapy, microwave therapy, active agents ozone, antibiotics, antimicrobials, or the like.

Alternatively or additionally, a wound may be treated using topical negative pressure or traditional advanced wound care, which is not aided by the using of applied negative pressure (may also be referred to as non-negative pressure therapy).

Advanced wound care may include use of an absorbent dressing, an occlusive dressing, use of an antimicrobial or debriding agents in a wound dressing or adjunct, a pad (for example, a cushioning or compressive therapy, such as stockings or bandages), or the like.

In some arrangements, treatment of such wounds can be performed using traditional wound care, wherein a dressing can be applied to the wound to facilitate and promote healing of the wound.

Some arrangements relate to methods of manufacturing a wound dressing including providing a wound dressing as disclosed herein.

The wound dressings that may be utilized in conjunction with the disclosed technology include any known dressing in the art. The technology is applicable to negative pressure therapy treatment as well as non-negative pressure therapy treatment.

In some arrangements, a wound dressing includes one or more absorbent layer(s). The absorbent layer may be a foam or a superabsorbent. In some arrangements, wound dressings may include a dressing layer including a polysaccharide or modified polysaccharide, a polyvinylpyrrolidone, a polyvinyl alcohol, a polyvinyl ether, a polyurethane, a polyacrylate, a polyacrylamide, collagen, or gelatin or mixtures thereof. Dressing layers including the polymers listed are known in the art as being useful for forming a wound dressing layer for either negative pressure therapy or non-negative pressure therapy.

In some arrangements, the polymer matrix may be a polysaccharide or modified polysaccharide. In some arrangements, the polymer matrix may be a cellulose. Cellulose material may include hydrophilically modified cellulose such as methyl cellulose, carboxymethyl cellulose (CMC), carboxymethyl cellulose (CEC), ethyl cellulose, propyl cellulose, hydroxyethyl cellulose, hydroxypropyl cellulose, hydroxypropylmethyl cellulose, carboxyethyl sulphonate cellulose, cellulose alkyl sulphonate, or mixtures thereof.

In certain arrangements, cellulose material may be cellulose alkyl sulphonate. The alkyl moiety of the alkyl sulphonate substituent group may have an alkyl group having 1 to 6 carbon atoms, such as methyl, ethyl, propyl, or butyl. The alkyl moiety may be branched or unbranched, and hence suitable propyl sulphonate substituents may be 1- or 2-methyl-ethylsulphonate. Butyl sulphonate substituents may be 2-ethyl-ethylsulphonate, 2,2-dimethyl-ethylsulphonate, or 1,2-dimethyl-ethylsulphonate. The alkyl sulphonate substituent group may be ethyl sulphonate. The cellulose alkyl sulphonate is described in WO10061225, US2016/114074, US2006/0142560, or US 5,703,225.

Cellulose alkyl sulfonates may have varying degrees of substitution, the chain length of the cellulose backbone structure, and the structure of the alkyl sulfonate substituent. Solubility and absorbency are largely dependent on the degree of substitution: as the degree of substitution is increased, the cellulose alkyl sulfonate becomes increasingly soluble. It follows that, as solubility increases, absorbency increases.

In some arrangements, a wound dressing also includes a top or cover layer. The thickness of the wound dressing disclosed herein may be between 1 to 20, or 2 to 10, or 3 to 7 mm.

Wound Dressing for Standalone Use

In some arrangements, the disclosed technology may be used in conjunction with a non-negative pressure dressing. A non-negative pressure wound dressing suitable for providing protection at a wound site may comprise:

an absorbent layer for absorbing wound exudate and an obscuring element for at least partially obscuring a view of wound exudate absorbed by the absorbent layer in use.

The obscuring element may be partially translucent. The obscuring element may be a masking layer. The non-negative pressure wound dressing may further include a region in or adjacent the obscuring element for allowing viewing of the absorbent layer. For example, the obscuring element layer may be provided over a central region of the absorbent layer and not over a border region of the absorbent layer. In some arrangements, the obscuring element is of hydrophilic material or is coated with a hydrophilic material. The obscuring element may include a three-dimensional knitted spacer fabric. The spacer fabric is known in the art and may include a knitted spacer fabric layer. The obscuring element may further include an indicator for indicating the need to change the dressing.

In some arrangements, the obscuring element is provided as a layer at least partially over the absorbent layer, further from a wound site than the absorbent layer in use.

The non-negative pressure wound dressing may further include a plurality of openings in the obscuring element for allowing fluid to move therethrough. The obscuring element may comprise, or may be coated with, a material having size-exclusion properties for selectively permitting or preventing passage of molecules of a predetermined size or weight.

The obscuring element may be configured to at least partially mask light radiation having wavelength of 600 nm and less. The obscuring element may be configured to reduce light absorption by 50% or more. The obscuring element may be configured to yield a CIE L* value of 50 or more, and optionally 70 or more. In some arrangements, the obscuring element may be configured to yield a CIE L* value of 70 or more.

In some arrangements, the non-negative pressure wound dressing may further include at least one of a wound contact layer, a foam layer, an odor control element, a pressure-resistant layer and a cover layer.

In some arrangements, the cover layer is present, and the cover layer is a translucent film. Typically, the translucent film has a moisture vapour permeability of 500g/m2/24hours or more. The translucent film may be a bacterial barrier.

In some arrangements, the non-negative pressure wound dressing as disclosed herein includes the wound contact layer and the absorbent layer overlies the wound contact layer. The wound contact layer carries an adhesive portion for forming a substantially fluid tight seal over the wound site.

The non-negative pressure wound dressing as disclosed herein may include the obscuring element and the absorbent layer being provided as a single layer.

In some arrangements, the non-negative pressure wound dressing disclosed herein includes the foam layer, and the obscuring element is of a material including components that may be displaced or broken by movement of the obscuring element.

In some arrangements, the non-negative pressure wound dressing includes an odor control element, and in another arrangement the dressing does not include an odor control element. When present, the odor control element may be dispersed within or adjacent the absorbent layer or the obscuring element. Alternatively, when present the odor control element may be provided as a layer sandwiched between the foam layer and the absorbent layer.

In some arrangements, the disclosed technology for a non-negative pressure wound dressing includes a method of manufacturing a wound dressing, including: providing an absorbent layer for absorbing wound exudate; and providing an obscuring element for at least partially obscuring a view of wound exudate absorbed by the absorbent layer in use.

In some arrangements, the non-negative pressure wound dressing is may be suitable for providing protection at a wound site, including: an absorbent layer for absorbing wound exudate; and a shielding layer provided over the absorbent layer, and further from a wound-facing side of the wound dressing than the absorbent layer. The shielding layer may be provided directly over the absorbent layer. In some arrangements, the shielding layer includes a three-dimensional spacer fabric layer.

The shielding layer increases the area over which a pressure applied to the dressing is transferred by 25% or more or the initial area of application. For example the shielding layer increases the area over which a pressure applied to the dressing is transferred by 50% or more, and optionally by 100% or more, and optionally by 200% or more.

The shielding layer may include 2 or more sub-layers, wherein a first sub-layer includes through holes and a further sub-layer includes through holes and the through holes of the first sub-layer are offset from the through holes of the further sub-layer.

The non-negative pressure wound dressing as disclosed herein may further include a permeable cover layer for allowing the transmission of gas and vapor therethrough, the cover layer provided over the shielding layer, wherein through holes of the cover layer are offset from through holes of the shielding layer. The non-negative pressure wound dressing may be suitable for treatment of pressure ulcers.

A more detailed description of the non-negative pressure dressing disclosed hereinabove is provided in WO2013007973.

In some arrangements, the non-negative pressure wound dressing may be a multi-layered wound dressing including: a fibrous absorbent layer for absorbing exudate from a wound site; and a support layer configured to reduce shrinkage of at least a portion of the wound dressing.

In some arrangements, the multi-layered wound dressing disclosed herein, further includes a liquid impermeable film layer, wherein the support layer is located between the absorbent layer and the film layer.

The support layer disclosed herein may include a net. The net may include a geometric structure having a plurality of substantially geometric apertures extending therethrough. The geometric structure may for example include a plurality of bosses substantially evenly spaced and joined by polymer strands to form the substantially geometric apertures between the polymer strands.

The net may be formed from high density polyethylene. The apertures may have an area from 0.005 to 0.32 mm2. The support layer may have a tensile strength from 0.05 to 0.06 Nm. The support layer may have a thickness of from 50 to 150 $\mu$m.

In some arrangements, the support layer is located directly adjacent the absorbent layer. Typically, the support layer is bonded to fibers in a top surface of the absorbent layer. The support layer may further include a bonding layer, wherein the support layer is heat laminated to the fibers in the absorbent layer via the bonding layer. The bonding layer may include a low melting point adhesive such as ethylene-vinyl acetate adhesive.

In some arrangements, the multi-layered wound dressing disclosed herein further includes an adhesive layer attaching the film layer to the support layer.

In some arrangements, the multi-layered wound dressing disclosed herein further includes a wound contact layer located adjacent the absorbent layer for positioning adjacent a wound. The multi-layered wound dressing may further include a fluid transport layer between the wound contact layer and the absorbent layer for transporting exudate away from a wound into the absorbent layer.

A more detailed description of the multi-layered wound dressing disclosed hereinabove is provided in GB patent application filed on 28 October 2016 with application number GB 1618298.2.

In some arrangements, the disclosed technology may be incorporated in a wound dressing including a vertically lapped material including: a first layer of an absorbing layer of material, and a second layer of material, wherein the first layer being constructed from at least one layer of non-woven textile fibers, the non-woven textile fibers being folded into a plurality of folds to form a pleated structure. In some arrangements, the wound dressing further includes a second layer of material that is temporarily or permanently connected to the first layer of material. Typically the vertically lapped material has been slitted.

In some arrangements, the first layer has a pleated structure having a depth determined by the depth of pleats or by the slitting width. The first layer of material may be a moldable, lightweight, fiber-based material, blend of material or composition layer.

The first layer of material may include one or more of manufactured fibers from synthetic, natural or inorganic polymers, natural fibers of a cellulosic, proteinaceous or mineral source.

The wound dressing may include two or more layers of the absorbing layer of material vertically lapped material stacked one on top of the other, wherein the two or more layers have the same or different densities or composition.

The wound dressing may in some arrangements include only one layer of the absorbing layer of material vertically lapped material.

The absorbing layer of material is a blend of natural or synthetic, organic or inorganic fibers, and binder fibers, or bicomponent fibers typically PET with a low melt temperature PET coating to soften at specified temperatures and to act as a bonding agent in the overall blend.

In some arrangements, the absorbing layer of material may be a blend of 5 to 95 % thermoplastic polymer, and 5 to 95 wt % of a cellulose or derivative thereof. In some arrangements, the wound dressing disclosed herein has a second layer includes a foam or a dressing fixative. The foam may be a polyurethane foam. The polyurethane foam may have an open or closed pore structure. The dressing fixative may include bandages, tape, gauze, or backing layer.

In some arrangements, the wound dressing as disclosed herein includes the absorbing layer of material connected directly to a second layer by lamination or by an adhesive, and the second layer is connected to a dressing fixative layer. The adhesive may be an acrylic adhesive, or a silicone adhesive.

In some arrangements, the wound dressing as disclosed herein further includes layer of a superabsorbent fiber, or a viscose fiber or a polyester fiber.

In some arrangements, the wound dressing as disclosed herein further includes a backing layer. The backing layer may be a transparent or opaque film. Typically the backing layer includes a polyurethane film (typically a transparent polyurethane film).

A more detailed description of the multi-layered wound dressing disclosed hereinabove is provided in GB patent applications filed on 12 December 2016 with application number GB1621057.7; and 22 June 2017 with application number GB 1709987.0.

In some arrangements, the non-negative pressure wound dressing may include an absorbent component for a wound dressing, the component including a wound contacting layer including gel forming fibers bound to a foam layer, wherein the foam layer is bound directly to the wound contact layer by an adhesive, polymer based melt layer, by flame lamination or by ultrasound.

The absorbent component may be in a sheet form. The wound contacting layer may include a layer of woven or non-woven or knitted gel forming fibers. The foam layer may be an open cell foam, or closed cell foam, typically an open cell foam. The foam layer is a hydrophilic foam.

The wound dressing may include the component that forms an island in direct contact with the wound surrounded by periphery of adhesive that adheres the dressing to the wound. The adhesive may be a silicone or acrylic adhesive, typically a silicone adhesive. The wound dressing may be covered by a film layer on the surface of the dressing furthest from the wound.

A more detailed description of the wound dressing of this type hereinabove is provided in EP2498829.

In some arrangements, the non-negative pressure wound dressing may include a multi layered wound dressing for use on wounds producing high levels of exudate, characterized in that the dressing including: a transmission layer having an MVTR of at least 300 gm2/24 hours, an absorbent core including gel forming fibers capable of absorbing and retaining exudate, a wound contacting layer including gel forming fibers which transmits exudate to the absorbent core and a keying layer positioned on the absorbent core, the absorbent core and wound contacting layer limiting the lateral spread of exudate in the dressing to the region of the wound.

The wound dressing may be capable of handling at least 6g (or 8g and 15g) of fluid per 10cm2 of dressing in 24 hours. The wound dressing may include gel forming fibers that are chemically modified cellulosic fibers in the form of a fabric. The fibers may include carboxymethylated cellulose fibers, typically sodium carboxymethylcellulose fiber. The wound dressing may include a wound contact layer with a lateral wicking rate from 5mm per minute to 40mm per minute. The wound contact layer may have a fiber density between 25gm2 and 55gm2, such as 35gm2. The absorbent core may have an absorbency of exudate of at least 10g/g, and typically a rate of lateral wicking of less the 20mm per minute. The absorbent core may have a blend in the range of up to 25% cellulosic fibers by weight and 75% to 100% gel forming fibers by weight.

Alternatively, the absorbent core may have a blend in the range of up to 50% cellulosic fibers by weight and 50% to 100% gel forming fibers by weight. For example the blend is in the range of 50% cellulosic fibers by weight and 50% gel forming fibers by weight. The fiber density in the absorbent core may be between 150gm2 and 250gm2, or about 200 gm2.

The wound dressing when wet may have shrinkage that is less than 25% or less than 15% of its original size/dimension. The wound dressing may include a transmission layer and the layer is a foam. The transmission layer may be a polyurethane foam laminated to a polyurethane film. The wound dressing may include one or more layers selected from the group including a soluble medicated film layer; an odor-absorbing layer; a spreading layer and an additional adhesive layer. The wound dressing may be 2mm and 4mm thick. The wound dressing may be characterized in that the keying layer bonds the absorbent core to a neighboring layer. In some arrangements, the keying layer may be positioned on either the wound facing side of the absorbent core or the non-wound facing side of the absorbent core. In some arrangements, the keying layer is positioned between the absorbent core and the wound contact layer. The keying layer is a polyamide web.

A more detailed description of the wound dressing of this type hereinabove is provided in EP1718257.

In some arrangements, the non-negative pressure wound dressing may be a compression bandage. Compression bandages are known for use in the treatment of oedema and other venous and lymphatic disorders, for example, of the lower limbs.

Compression bandage systems typically employ multiple layers including a padding layer between the skin and the compression layer or layers. The compression bandage may be useful for wounds such as handling venous leg ulcers.

The compression bandage in some arrangements may include a bandage system including an inner skin facing layer and an elastic outer layer, the inner layer including a first ply of foam and a second ply of an absorbent nonwoven web, the inner layer and outer layer being sufficiently elongated so as to be capable of being wound about a patient's limb. A compression bandage of this type is disclosed in WO99/58090.

In some arrangements, the compression bandage system comprises: a) an inner skin facing, elongated, elastic bandage including: (i) an elongated, elastic substrate, and (ii) an elongated layer of foam, said foam layer being affixed to a face of said substrate and extending 33% or more across said face of substrate in transverse direction and 67% or more across said face of substrate in longitudinal direction; and b) an outer, elongated, self-adhering elastic bandage; said bandage having a compressive force when extended; wherein, in use, said foam layer of the inner bandage faces the skin and the outer bandage overlies the inner bandage. A compression bandage of this type is disclosed in WO2006/110527.

In some arrangements other compression bandage systems such as those disclosed in US 6,759,566 and US 2002/0099318.

Another example of a multi-layer wound dressing is the ALLEVYN dressing, including ALLEVYN Life and ALLEVYN Gentle Border dressings, available from Smith & Nephew, which include a moist wound environment dressing that is used to treat the wound.

Monitoring and/or Therapy

Disclosed herein are arrangements of a monitoring and/or therapy apparatuses and methods. An apparatus can have one or more strain sensors (also referred to herein as a strain gauge) that, in any arrangements, can have an electroactive polymer that can be used to measure at least one of a motion, a range of motion, a shape, and/or a stiffness of joints or body parts of a human, animal, or other living user. The strain sensors can be capacitive strain sensors.

Any arrangements (which is meant to include, without limitation, any arrangements disclosed and/or incorporated by reference herein, including any combination of any of the individual components, materials, features, shapes, or other details of any of the arrangements disclosed herein, and any variants of such that would be readily apparent or obvious to one of ordinary skill in the art, or other arrangements that are within the scope of any of the claim herein) of monitoring and/or therapy apparatus disclosed herein can have a contact layer (or substrate) configured to be attached to a tissue of a user, a strain sensor coupled with the contact layer, and electronic circuitry that can be electronically coupled with the strain sensor. The electronic circuitry can be configured to determine a level of at least one of an elongation or a curvature of the strain sensor based on changes in, for instance, the capacitance, impedance, resistance, or the like of the strain sensor. In any arrangements disclosed herein, the strain sensor can be configured to provide data to the electronic circuitry related to an amount of the curvature of the contact layer adjacent to the strain sensor.

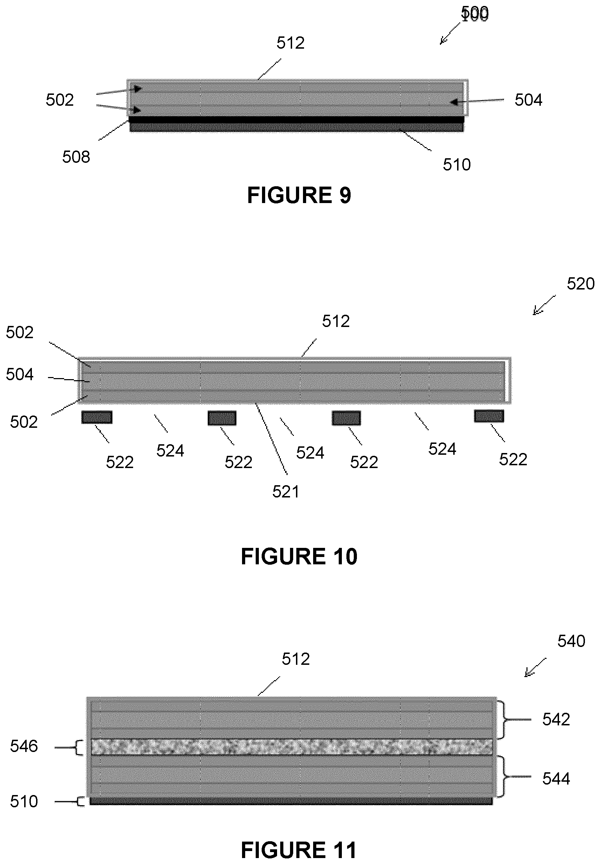

Figure 9 illustrates a side view of an arrangement of a strain sensor 500 that can be used with any of the monitoring and/or therapy apparatus arrangements disclosed herein. With reference to Figure 9, for example and without limitation, the strain sensor 500 can be a strain sensor (also referred to herein as a strain gauge). The strain sensor can be configured such that capacitance of the strain sensor 500 changes responsive to the strain sensor 500 being elongated and/or curved. The strain sensor 500 can have two or more electrodes 502 (two being shown in Figure 9) and a dielectric layer 504 positioned between the two electrodes 502. The two electrodes can comprise a first and a second electrode. The dielectric layer can comprise an electroactive polymer or any other suitable dielectric material. If an electroactive polymer is used, then the potential generated by stretching the sensor can be used to generate a signal. In some arrangements, using an electroactive polymer can allow for a 'passive' system where the stretching or elongation of the sensor and, hence, the electroactive polymer can create electricity that can be harvested to power the device or to create a signal to indicate the strain. Therefore, any arrangements disclosed herein comprising an electroactive polymer can be configured to operate battery-less (i.e., without the use of a battery) for at least the sensor. In some arrangements, the electroactive polymer can also be used to power the electronic circuitry.

In some arrangements, the dielectric layer or any other layer of the sensor can include or be made from ceramic, glass, plastics, PTFE, polyethylene, the oxides of various metals, and/or any other suitable materials or combinations of the foregoing. Additionally, in any arrangements, the electrodes can include or be made from carbon, carbon nanotubes, graphene, titanium, silver, palladium, and/or any other suitable materials or combinations of the foregoing.

In some arrangements, the strain sensor can be integrated with a contact layer or substrate, or the strain sensor can have adhesive on at least a portion of a surface of the strain sensor that is in contact with the contact layer. With reference to Figure 9, a contact layer 510, such as an adhesive layer 510, can be positioned on a bottom surface of the strain sensor 500. The adhesive can be an acrylic adhesive or any other suitable adhesive. Further, an isolation layer, film, or substrate 508 can be positioned between the electrodes and a user's tissue surface or the contact layer to electrically isolate the electrodes and/or the electroactive polymer from the user. The electrodes and electroactive polymer can further be encapsulated or covered with an isolation layer, film, or substrate 512 to electrically isolate the electrodes and/or the electroactive polymer from the user and to protect the electrodes and/or electroactive polymer. In some arrangements, the substrate 508 can be the same material as the substrate 512 that can cover the electrodes, etc.

Strain Sensor Details

In any arrangements disclosed herein, with reference to Figure 10, the strain sensor 520 can have a first adhesive segment 522 attached to a first surface 121 of the strain sensor 520 (such as the electrode layer 502), a second adhesive segment 522 attached to the same first surface 121 of the strain sensor 520, and a space 524 between the first and second adhesive segments 522, wherein the space does not include or have any adhesive. In some arrangements, the strain sensor can have any desired number of adhesive segments 522, including three or more adhesive segments, four or more adhesive segments (as is shown in Figure 10), or from three to five adhesive segments, or more, along a surface of the strain sensor.

As described herein, the dielectric layer of any of the strain sensor arrangements disclosed herein can comprise an electroactive polymer material. Further, any arrangements of the strain sensor can be encased in a silicone isolator layer or material. An adhesive (such as, but not limited to, a silicone adhesive) can be used between the strain sensor and/or a contact layer or other material, substrate, or object that the strain sensor is coupled with to ensure intimate contact under strain when the tissues move beneath the strain sensor or the contact layer, or otherwise.

In some arrangements, the strain gauge or sensor can include a dielectric polymer sandwiched between two (or more) electrodes. As the sensor is elongated or otherwise deformed, distance between the electrodes changes (for example, gets smaller), causing the electrical capacitance to change. The change in the capacitance can be indicative of elongation of the sensor.

In some arrangements, the strain gauge can include an insulating flexible backing which supports a metallic foil pattern. The strain gauge can be attached to an enclosure (also referred to herein as a housing) or the contact layer by a suitable adhesive. As the load-measuring device is deformed, the foil is deformed, causing the electrical capacitance, inductance, or resistance to change. This change can be measured, for example, by a bridge circuit (such as, Wheatstone bridge circuit), LCR meter, or the like, and can be related to the strain by the gauge factor quantity.

In any arrangements, semiconductor strain gauges (such as, piezoresistors), nanoparticle-based strain gauges, mercury-in-rubber strain gauges, microscale strain gauges, piezoresistive strain gauges, integrated optical ring resonators, piezoelectric strain gauges, vibrating wire strain gauges, fiber optic strain gauges, and/or other capacitive strain gauges can be used. Fiber optic strain gauges can also be employed to measure strain along an optical fiber. Measurements can be distributed along the fiber, or taken at predetermined points on the fiber.

Any sensors disclosed herein can be any of a wide ranging variety of shapes and may be perforated, provided that the perforations are sealed to protect the electrodes, and can be nonperforated.

Double Layer Strain Sensor

With reference to the arrangement of the strain sensor 540 illustrated in Figures 3 and 4 as an example, any arrangements of the strain sensor or the monitoring and/or therapy apparatus disclosed herein can have double layer (or more) strain sensor having a first strain gauge (or sensor) 542 positioned over, and in at least approximate alignment with, a second strain gauge (or sensor) 544. In some arrangements, a spacer layer 544 can be positioned between the first strain gauge 542 and the second strain gauge (or sensor) 544. In some arrangements, the first capacitive strain gauge 542 can be positioned directly over (i.e., in direct contact with, or with an adhesive layer therebetween) the second strain gauge 544. One or more of the first or second strain gauges can be capacitive strain gauges.

The strain sensor 540 can be configured such that a capacitance of the first strain gauge 542 changes responsive to the first strain gauge 542 being at least one of elongated and curved, and a capacitance of the second strain gauge 544 changes responsive to the second strain gauge 544 being at least one of elongated and curved. In any arrangements, the first strain gauge 542 can be positioned above the second strain gauge 544 and can be coupled with the second strain gauge 544 in alignment with the second strain gauge 544. In this configuration, the strain sensor 540 can be used to calculate a difference between an elongation of the first strain gauge and an elongation of the second strain gauge to determine an amount of an elongation, a curvature, or an elongation and a curvature of the contact layer.

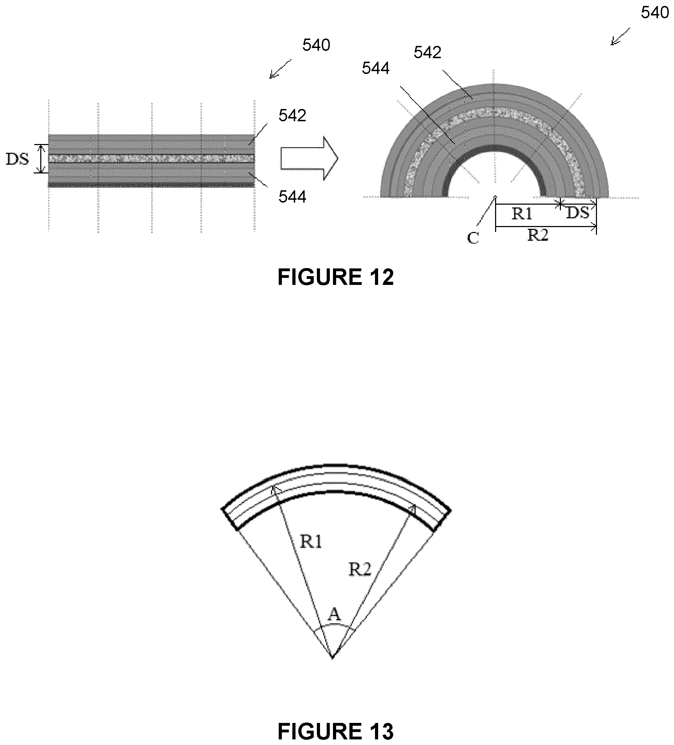

For example, with reference to Figure 12, when the strain sensor is bent or deformed in a curved state (shown in the right of Figure 12), wherein the first strain gauge 542 is firmly attached to or coupled with and has approximately the same relaxed-state length as the second strain gauge 544, the first strain gauge 542 must elongate or stretch more than the second strain gauge 544 due to the curvature of the strain sensor 540 when the strain sensor is being bent. Based on the difference in elongation of the first and second strain gauges, the apparatus can determine a radius or degree of curvature of the strain sensor to determine a degree or radius of curvature of the tissue or joint adjacent to or under the strain sensor.

In some arrangements, a strain sensor comprising the first and second strain gauges can be configured to bend in normal use so that the strain sensor has an approximately constant radius. For example and without limitation, in this arrangement (i.e., if the first strain gauge is fixed in alignment over the second strain gauge), if both sensors experience the same or substantially similar change in elongation, this will indicate that the strain sensor is undergoing only elongation and no or substantially no curvature. Further, knowing a distance between a center plane (which can be a neutral plane of each strain gauge) of each of the first and second strain gauges (i.e., the distance from a mid-plane of the first strain gauge to a mid-plane of the second strain gauge, as represented by DS in Figure 12), a radius (represented by R1 in Figure 12) from the center point or axis of the curve (represented by C in Figure 12) to the first strain gauge 542 will be greater than a radius (represented by R2 in Figure 12) from the center point or axis of the curve to the second strain gauge 544 by the known or calculable value of DS. The strain gauge that is further away from the center point or axis C (such as, the first strain gauge 542) will elongate more during bending than the strain gauge that is closer to the center point or axis of the curve (such as, the second strain gauge 544).

In this arrangement, the elongation (i.e., the change in the length of the sensor from the relaxed state to the strained state) can be determined for each of the first and the second strain gauges. If there is a substantial difference in the elongation of the first strain gauge and the second strain gauge, this will be indicative of the strain sensor (and, hence, the tissue beneath the strain sensor) being deformed in a curved manner and a joint adjacent to the strain sensor being bent or rotated. In this state, the angle of the strain sensor in the bent state (represented by A in Figure 13) can be determined using the following equation:

$$(1) \quad A = 180 \cdot L1/(\pi \cdot R1)$$

(where A is the angle in degrees)

$$(2) \quad A = 180 \cdot L2/(\pi \cdot R2)$$

(where A is the angle in degrees)

$$(3) \quad R1 = R2 - DS$$

(again, where DS is the distance from a mid-plane of the first strain gauge to a mid-plane of the second strain gauge)

Using these equations, R1 can be determined according to the following equation:

$$(4) \quad R2 = (L2 \cdot DS)/(L2-L1)$$

Because L1, L2, and DS will be known or calculable values from the strain sensor, R2 can be determined from equation (4) above. With R2 and DS known, A (i.e., the angle of the strain sensor in the bent state) can be determined using equation (2) above.

As described herein, any arrangements of the strain sensor disclosed herein can further have a spacer layer (such as spacer layer 546) between the first strain gauge 542 and the second strain gauge 544, wherein the spacer layer can be configured to increase a difference between an elongation of the first strain gauge and an elongation of the second strain gauge during bending of the strain sensor. The apparatus of any arrangements disclosed herein can further have a non-continuous spacer layer between the first strain gauge and the second strain gauge, wherein the non-continuous spacer layer can be configured to cause the strain sensor to perform like multiple discrete strain sensors rather than one continuous strain sensor. For example and without limitation, the strain sensor can further have a non-continuous spacer layer between the first strain gauge and the second strain gauge, wherein the spacer layer can be configured to decrease a stiffness of the strain sensor. For example, the spacer layer can be formed in strips extending transverse to a length of the strain sensor with open spaces between the plurality of strips. Some arrangements can have five or more lateral strips, or from three to ten or more lateral strips, or from four to 6 lateral strips along a length of the strain sensor between the first and the second strain gauges.

## Multiple Sensors

In any arrangements disclosed herein, the at least one strain sensor can have a first strain sensor and a second strain sensor spaced apart from the first strain sensor (wherein any of the strain sensors is a single strain sensor or a stacked strain sensor). The second strain sensor can be parallel to the first strain sensor, in line with the first strain sensor, spaced apart and at an oblique angle relative to the first strain sensor, or otherwise. For example and without limitation, an apparatus can have two, three, four, or more, or from two to five, or from three to four, strain sensors in series, in parallel, or in any desired arrangement (including, without limitation, any of the arrangements described herein).

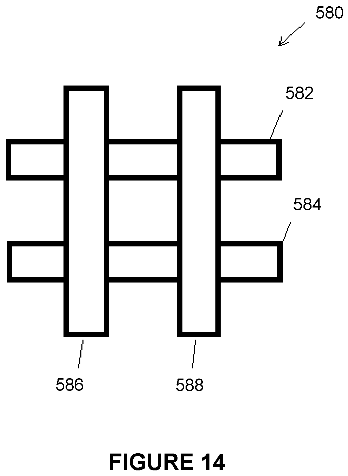

Figure 14 illustrates another arrangement of a strain sensor 580 have a first strain sensor 582 and a second strain sensor 584 in a spaced apart but parallel arrangement, a third strain sensor 586 and a fourth strain sensor 588 also in a spaced apart but parallel arrangement. The third and fourth strain sensors 586, 588 can be perpendicular to the first and the second strain sensors 582, 584. The strain sensor 580 can be used in any of the arrangements of the monitoring and/or therapy apparatus disclosed herein, and can have any of the features, materials, components, or other details of any of the other sensor arrangements disclosed herein.

The crisscross arrangement of the sensor 580 can be used to determine elongation and/or curvature of the skin surface and/or joint in multiple directions, and can be used to determine twisting in the tissue or joint adjacent to the sensor, which may be beneficial for joint characterization. For example and without limitation, the strain sensor 580 can be used to determine the elongation and/or curvature of the skin surface and/or joint along at least a lengthwise direction of the individual sensors or gauges. Additionally, in some arrangements, elongation and/or curvature can be determined along vectors that are oblique to the lengthwise directions of the individual sensors or gauges. Therefore, some arrangements can be configured to map strain/curvature in at least two axes, for which the data may be attributable to local vectors. Strain could be identified to occur at a particular part of the sensor, and therefore specific part of a joint/skin, providing insight into the mechanical stress that occurs and what specific part/tissues of the body is being affected.

## Electronic Circuitry

As described herein, any arrangements of the monitoring and/or therapy apparatus disclosed herein can have electronic circuitry electronically coupled with the strain sensors. The electronic circuitry can have machine-readable memory and a source of power for at least the electronic circuitry. In any arrangements disclosed herein, the at least one strain sensor and/or any portion of, or all of, the electronic circuitry can be positioned on or can be printed on the contact layer, including on an edge portion of the contact layer. In any arrangements disclosed herein, any or all components of the electronic circuitry can be coupled with the contact layer, or can be spaced apart from the contact layer.

The electronic circuitry of any arrangements disclosed herein can include one or more indicators, such as visual, audible, tactile, haptic, or the like to indicate a status of the device. For example, a light emitting diode (LED) visual indicator can be used to indicate one more conditions of the sensor or the apparatus.

In some cases, a substantially non-extensible or non-stretchable coating can be used to at least partially shield or protect the electronic circuitry from stress or strain, coming into contact with fluids, or the like. This coating can be one or more of a suitable adhesive, epoxy, polyester, polyimide, polyamide, PET, PBT, or another type of material with a high Young's modulus, such as Dymax 1901-M or 9001-E Dymax. The coating can be thin, such as about 100 microns thick, less than about 100 microns thick, or more than about 100 microns thick. The coating can be applied and cured using one or more of UV, light, or thermal curing. In some arrangements, the electronic circuitry can include a substantially flexible circuit board.

## Source of Power

In any arrangements disclosed herein, the apparatus can have a source of power for at least the electronic circuitry. The source of power can be located separate from the electronics circuitry, or coupled with the electronics circuitry, or otherwise. In some arrangements, the source of power can be coupled with or supported by the contact layer. In any arrangements disclosed herein, the source of power can have one or more flexible batteries and can be positioned on the contact layer. The source of power can be rechargeable (such as a lithium ion battery) or replaceable, or can be permanently coupled with the apparatus so as to not be replaceable.

## Communications Devices

Any of the arrangements of the apparatus disclosed herein can have a wired or wireless communications device electronically coupled with the electronic circuitry. The wireless communications device can be configured to transmit a signal containing an angle and/or a range of movement of the joint. Further, in any arrangements, the wireless communications device can be configured to transmit a signal containing at least one of a measurement of motion or a plurality of measurements of motion, a measurement or plurality of measurements of range of motion or movement (ROM), a measurement or plurality of measurements of shape or stiffness of body parts, or one or more measurements to monitor patient compliance (i.e., whether or not patients engaging in physiotherapy/stretch exercises), and/or the extent of compliance. In some arrangements, the apparatus can have a bluetooth communications device electronically coupled with the electronic circuitry.

The wired or wireless communications device can be configured to transmit information to a separate computing device, such as a computer (for example, clinician's computer), server, mobile computing device, pager, monitoring unit hospital monitoring system, or the like. In any arrangements, the information recorded or saved by the apparatus, and/or transmitted by the apparatus, can include strain sensor data (including maximum, minimum, and average values over a duration of time), range of movement information (including maximum, minimum, and average values over a duration of time), joint angle information (including maximum, minimum, and average values over a duration of time), usage data, alarm conditions, and/or any of the other parameters or characteristics described herein.

The communications device can transmit the information via one or more of Near Field Communication (NFC), BluetoothTM, BluetoothTM Low Energy, Zigbee, radio waves, Wi-Fi, or the like. The communications device can, for example and in some arrangements, communicate and pair with other devices (such as, without limitation, a smart phone, a tablet computer, or a desktop computer) and transmit the information in an encrypted or protected format. Additionally, in any arrangements disclosed herein, the apparatus and/or the communications device can also be configured to receive signals or information including, for example and without limitation, operating instructions for the device and/or data from an outside source. For example, the device can be configured to receive a signal that can cause the apparatus to trigger an alarm on the device, cause the device to enter a state of hibernation or lower power, or cause the device to change a frequency of data collection, or some other operating parameter of the device.

Housing

In some arrangements, the electronic circuitry, including the machine-readable memory and/or other components of the electronic circuitry, can be positioned within a housing that is separate from the contact layer, coupled with the contact layer, integrally formed with the contact layer, or otherwise. In any arrangements disclosed herein, the electronic circuitry can be positioned at least partially within a housing that is configured to be positionable at a distance from the contact layer so that the electronic circuitry can be spaced apart from the contact layer, or wherein the housing may be coupled with the contact layer. In any arrangements disclosed herein, the electronic circuitry can have a machine-readable memory storing executable instructions and being configured to record information related to an elongation of the at least one strain sensor.

Frequency of Data Collection

In any arrangements disclosed herein, the electronic circuitry is further configured to automatically collect data from the strain sensor at a predetermined frequency or (not according to the invention) over a predetermined length of time at a predetermined or varying frequency, or can be further configured to automatically gather readings from the strain sensor at a plurality of time intervals, and shorten a time interval in response to the strain sensor experiencing changes in the resistance at a higher frequency. The electronic circuitry can be further configured to increase a frequency of data collected from the strain sensor in response to the strain sensor experiencing at least one of a higher frequency or a greater magnitude of changes, for example, in the capacitance. In some applications, the apparatus can be used to measure motion, range of motion or movement (ROM), shape or stiffness of body parts, or to monitor patient compliance (i.e., whether or not patients engaging in physiotherapy/stretch exercises), and/or the extent of compliance.

In some arrangements, the apparatus can be configured to report daily values, such as max or min values, average values, or the like. Such information can be used to provide feedback to a user to inform the user regarding the progress of treatment, or regression of his/her/its condition.

Temperature Sensor

In any arrangements disclosed herein, the apparatus further has a temperature sensor in electrical communication with the electronic circuitry. The temperature sensor is configured to provide an electrical signal indicative of a temperature of the strain sensor and the electronic circuitry can be configured to calibrate the readings from the strain sensor based on the temperature of the strain sensor. The temperature sensor can be configured to enable the electronic circuitry to determine a temperature of the strain sensor or of the contact layer adjacent to the strain sensor.

The temperature sensor can be positioned on the substrate adjacent to the strain sensor, or multiple temperature sensors can be positioned on the substrate adjacent to multiple strain sensors. In any arrangements, the temperature sensor can be configured to provide to the electronic circuitry an electrical signal related to at least one of a temperature of the strain sensor or a temperature of a region of the substrate adjacent to the strain sensor. In some arrangements, the temperature sensor can be integrated with the strain sensor. The electronic circuitry can be further configured to calibrate readings from the strain sensor based on the temperature of the strain sensor.

In some arrangements, the temperature reading or data from the temperature sensor can be used to normalize the data from the strain gauge to correct or adjust the strain gauge readings for temperature effects. For example, in some arrangements, the system can be configured to apply a correction factor to the strain calculation to account for the thermal effects. In some arrangements, the data from the strain gauge could be normalized against, or to take into account, the known temperature. For example, a different strain profile associated with a temperature value or range can be used. In one example, at 25 degrees, a strain profile can be A-B, at 26 degrees, the profile can be C-D, or the like where a certain strain related measurement will result in a different strain based on the particular profile associated with the temperature reading.

In any of the arrangements disclosed herein, the apparatus can further include a temperature sensor in electrical communication with the electronic circuitry configured to enable the electronic circuitry to determine a temperature of the strain sensor and/or of the contact layer adjacent to the strain sensor. An example of an application of this would be for identification of successful thermal coupling between tissue and the temperature sensor or other sensor and/or between the material of the contact layer or dressing component and the sensor. Some sensors require close coupling to generate accurate sensor data. If there is insufficient compression on or insufficient coupling of the compression/strain sensor, then the data from the temperature (or other) sensor may be erroneous. The use of multiple capacitive sensors in an array with associated temperature (or other) sensors can provide information and feedback regarding the level of coupling between the sensor and the underlying substrate (such as, tissue, contact layer, or other material), or identification of the boundary where acceptable coupling had been made. It would be expected that a temperature reading would be accepted as valid when the force identified on the strain/compression sensor had exceeded a set value (or was between specific values) for a set time. This can provide a determination of the accuracy of the sensor reading.

Without limitation, one application would be on thermal mapping socks/scales for thermal hotspot identification (and/or contralateral thermal comparison). This can be used as a preventative measure for identification of tissue damage. The elastomeric sensors could also be used to identify the boundaries of the tissue that is resting against a surface, if using an ostensibly planar surface (such as, scales) or the acceptable measurement boundaries on socks. This could be used to identify the boundaries of acceptably coupled areas. **In** some embodiments, the implementation could be in a linear plate or a sock configuration. **In** a sock configuration, the gauges could be used to also identify localised tension, using this to detect a change in shape or to determine if the sock is twisted.

**In** any arrangements disclosed herein, the strain gauge can be an elastomeric strain gauge that can be used in combination with one or more temperature sensors. These arrangements can be configured to identify tissue and/or appendage position (for example, the position of the foot or other portion of the body) or loading for a temperature mapping product. Additionally, some arrangements can be configured for use for contralateral temperature measurement for identification of diabetic foot ulcers, and also to provide feedback and data to identify of the foot boundary (i.e., location of the periphery of the foot).

Alarm

Any arrangements of the apparatuses disclosed herein can further have an alarm in electronic communication with the electronic circuitry. The electronic circuitry can be further configured to activate the alarm in response to a capacitance, impedance, resistance, elongation, and/or curvature of any of the strain sensors (if more than one) satisfying a threshold capacitance, impedance, resistance so as to indicate a threshold elongation of the cover layer, or a threshold curvature of the cover layer, a threshold bend angle of the joint, and/or otherwise.

Shape

In any arrangements disclosed herein, the contact layer can be configured to have a shape which substantially matches a joint when the contact layer is in a relaxed state. In any arrangements disclosed herein, the contact layer can be configured to have a shape which substantially matches a joint when the contact layer is in a relaxed state, wherein the joint can be an elbow joint, a wrist joint, a knee joint, a neck joint, a spine, a finger joint, or an ankle joint. In any arrangements, the contact layer can have a tubular shape.

Applications of Any Arrangements Disclosed Herein

In some arrangements, the apparatus (which includes the sensor and electronic circuitry) can be used to measure a rate of change of strain and/or curvature to identify stiffness in a motion of a joint. In some arrangements, this can be augmented with a frequency spectrum (such as, via an FFT) on the value to identify muscle tremor. One or multiple sensors and electronic circuitry can also be applied a calf muscle to identify the operation and function of the calf-pump (for blood circulation).

Any arrangements of the apparatus can also be used for the identification of function of a muscle stimulation system (such as, in addition to the above, or in other stimulation system - allowing feedback to tailor the stimulation amplitude), and/or to identify Parkinson tremors under specific range of motions (and speeds) in order to eliminate the need for a daily test on an app.

In some arrangements, the double layer sensor or single sensors can be positioned laterally across the hip to identify hip extension/displacement and movement as a precursor to or post orthopaedic surgery. Additionally, the double layer sensor or single sensors can be used for measurement of hip distraction.

In some arrangements, the double layer sensor or single sensors can be positioned over a patient's kneecap or elbow or to identify motion of the knee or elbow. In addition, a third sensor (which may be of the same type, a strain gauge, or other), can be mounted towards the foot side of the knee (for example, without limitation, over the patella), that has partially free motion and a small weight or mass on a lower end of the strain sensor. In this configuration, the sensor/strain gauge will be strained if the sensor is in a vertical orientation, will experience no strain if the sensor is in a horizontal orientation, and will experience a degree of strain commensurate with an orientation angle of the sensor when the sensor is at an angle relative to the vertical orientation. As such, the relative orientation of the sensor or sensor array may be identified (such as, in order to differentiate between standing up and sitting with feet up). Any arrangements of the apparatuses disclosed herein may comprise a second or an additional sensor or sensors that can include a magnetometer, accelerometer, spirit level, tilt switch or other sensor(s) or any combination thereof.

Additionally, in some arrangements, the sensor can act as a variable capacitor that can be used as part of a tuning circuit. The value of the tuned frequency can be used to identify the strain on the sensor to allow a direct remote reading. Alternatively, the tuned frequency can receive only at that frequency and then broadcast back either at that frequency or, by using a transducer, at a different frequency. In either case, different sensors could be configured to run at different frequencies or to have a specific time-based pulse.

An alternative implementation could utilize one or more out of colored/polarized/lenticular material that changes color/polarization/paralax during elongation. This could be used in single or dual layer sensors and could allow identification of the output from each layer. This could be read by eye, by a reader (such as, app) or by a sensor within or associated with the strips.

Wound Application

In any arrangements disclosed herein, the contact layer can be formed from a substantially impermeable material and can be configured to be positioned over a wound. Further, the apparatus can have a source of negative pressure in fluid communication with a space under the contact layer, the source of negative pressure being configured to aspirate fluid from a wound over which the contact layer can be positioned. Any arrangements disclosed herein can further have a port in fluid communication with the contact layer configured to communicate a source of reduced pressure to a space between the contact layer and a wound. In some cases, the apparatus can provide additional or alternative forms of treatment, such as irrigation, ultrasound, heat and/or cold, neuro stimulation, or the like. In some cases, disclosed systems and methods can be used for wound monitoring without application of additional therapy. Systems and methods disclosed herein can be used in conjunction with a dressing, including with compression dressing, reduced pressure dressing, or the like.

Other Arrangements

In other arrangements, the apparatus can have a wound cover that can have an impermeable contact layer sized and configured to be attached to a skin surface of a user and to cover a wound, and a strain sensor configured to determine a strain by passing an electrical signal through the strain sensor. The apparatus of any arrangements disclosed herein can also have a machine-readable memory having stored thereon executable instructions, and a processor in communication with the machine-readable memory and the strain sensor. The strain sensor can be coupled with the contact layer and can be configured such that, for example, the capacitance of the strain sensor changes as the strain sensor is elongated or curved. Further, the processor can be configured to execute the instructions stored on the machine-readable memory to cause the processor to automatically determine the capacitance of the strain sensor at a predetermined frequency and, from the capacitance of the strain sensor, to determine a level of an amount of an elongation and/or a curvature of the contact layer adjacent to the strain sensor. Further, in any arrangements, the strain sensor can be configured to provide data to the processor related to an amount of the curvature of the contact layer adjacent to the strain sensor.

In other arrangements, the apparatus can have a wound cover having an impermeable contact layer sized and configured to be attached to a skin surface of a user and to cover a wound, and a strain sensor that can have a first strain sensor and a second strain sensor coupled with the contact layer. The strain sensor can be configured to provide data related to an amount of an elongation and/or a curvature of the contact layer adjacent to the strain sensor to a processor. Any arrangements can further have a machine-readable memory having stored thereon executable instructions, and a processor in communication with the machine-readable memory and the strain sensor, wherein the processor can be configured to execute the instructions stored on the machine-readable memory to cause the apparatus to automatically gather one or more readings from the strain sensor. The first strain sensor can be configured to provide data associated with an elongation of the contact layer associated with a stretching or a bending of the contact layer adjacent to the first strain sensor to the processor, and the second strain sensor can be configured to provide data associated with an elongation of the contact layer associated with a stretching or a bending of the contact layer adjacent to the second strain sensor to the processor.

Other Variations

Any of the systems and methods disclosed herein can be used with any one or more of features described in International Patent Application No. PCT/EP2019/065602, filed on June 13 2019.

Although certain arrangements described herein relate to pressure ulcers and/or wound dressings, systems and methods disclosed herein are not limited to pressure ulcer and/or wound dressing applications. Systems and methods disclosed herein are generally applicable to monitoring and/or treatment of a body part. In some cases, systems and methods disclosed herein may be applicable to monitoring of an object, such as a robotic limb.

Although this disclosure describes certain arrangements, it will be understood by those skilled in the art that many aspects of the methods and devices shown and described in the present disclosure may be differently combined and/or modified to form still further arrangements or acceptable examples. All such modifications and variations are intended to be included herein within the scope of this disclosure. Indeed, a wide variety of designs and approaches are possible and are within the scope of this disclosure. No feature, structure, or step disclosed herein is essential or indispensable. Moreover, while illustrative arrangements have been described herein, the scope of any and all arrangements having equivalent elements, modifications, omissions, combinations (for example, of aspects across various arrangements), substitutions, adaptations and/or alterations as would be appreciated by those in the art based on the present disclosure. While certain arrangements have been described, these arrangements have been presented by way of example only, and are not intended to limit the scope of protection. The scope of protection is defined by the claims.

Features, materials, characteristics, or groups described in conjunction with a particular aspect, arrangement, or example are to be understood to be applicable to any other aspect, arrangement or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The disclosure is not restricted to the details of any foregoing arrangements. The disclosure extends to any novel one, or any novel combination, of the features disclosed in this specification (including any accompanying claims, abstract and drawings), or to any novel one, or any novel combination, of the steps of any method or process so disclosed.

While certain arrangements have been described, these arrangements have been presented by way of example only, and are not intended to limit the scope of protection. Indeed, the novel methods and systems described herein may be embodied in a variety of other forms. Furthermore, various omissions, substitutions and changes in the form of the methods and systems described herein may be made. Those skilled in the art will appreciate that in some arrangements, the actual steps taken in the processes illustrated and/or disclosed may differ from those shown in the figures. Depending on the arrangement, certain of the steps described above may be removed, others may be added. For example, the actual steps and/or order of steps taken in the disclosed processes may differ from those shown in the figure. Depending on the arrangement, certain of the steps described above may be removed, others may be added. For instance, the various components illustrated in the figures may be implemented as software and/or firmware on a processor, controller, ASIC, FPGA, and/or dedicated hardware. Hardware components, such as processors, ASICs, FPGAs, and the like, can include logic circuitry.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some arrangements, the actual steps taken in the processes illustrated and/or disclosed may differ from those shown in the figures. Depending on the arrangement, certain of the steps described above may be removed, others may be added. Furthermore, the features and attributes of the specific arrangements disclosed above may be combined in different ways to form additional arrangements, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular arrangement. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain arrangements include, while other arrangements do not include, certain features, elements, and/or steps. Thus, such conditional language is not generally intended to imply that features, elements, and/or steps are in any way required for one or more arrangements or that one or more arrangements necessarily include logic for deciding, with or without user input or prompting, whether these features, elements, and/or steps are included or are to be performed in any particular arrangement. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain arrangements require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain arrangements, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

## Claims

1. A monitoring and/or therapy apparatus (200, 300, 320, 320'), comprising:

   A contact layer (202, 302, 322, 322') comprising a stretchable or substantially stretchable substrate configured to be attached to a user's joint;
   at least one strain sensor (500, 520, 540) coupled with the contact layer (202, 302, 322, 322');
   electronic circuitry (210) electronically coupled with the at least one strain sensor, the electronic circuitry (210) configured to determine an amount of an elongation of the at least one strain sensor based on a change in a resistance of the at least one strain sensor (500, 520, 540); and
   a source of negative pressure configured to be in fluid communication with a space under the contact layer (202, 302, 322, 322') and to supply negative pressure to the space;
   wherein:

   the at least one strain sensor (500, 520, 540) comprises a plurality of stretchable tracks (204, 304, 324, 324') positioned on the substrate;
   the resistance of the at least one strain sensor increases as at least some tracks of the plurality of stretchable tracks are elongated due to the contact layer being elongated;
   the electronic circuitry is configured to determine an angle and/or a range of movement of the joint responsive to changes in the resistance of the at least one strain sensor (500, 520, 540);
   wherein the electronic circuitry comprises a machine-readable memory configured to record information related to an elongation of the at least one strain sensor;
   the electronic circuitry is configured to automatically collect data from the strain sensor at a predetermined frequency; and
   the apparatus further comprises a temperature sensor, wherein the temperature sensor is configured to provide to the electronic circuitry an electrical signal related to a temperature of the strain sensor and/or a temperature of a region of the substrate adjacent to the strain sensor.

2. The apparatus of claim 1, wherein the plurality of stretchable tracks comprises conductive ink printed on the substrate.

3. The apparatus of claim 1 or claim 2, wherein the electronic circuitry is coupled with the contact layer or spaced apart from the contact layer.

4. The apparatus of any one of the preceding claims, wherein at least some of the electronic circuitry is printed directly on the contact layer.

5. The apparatus of any one of the preceding claims, wherein the electronic circuitry is positioned at least partially within a housing (310, 328) that is configured to be positionable at a distance from the contact layer so that the electronic circuitry is spaced apart from the contact layer, and wherein the housing (310, 328) may be coupled with the contact layer.

6. The apparatus of any one of the preceding claims, wherein the at least one strain sensor and/or the electronic circuitry is positioned on or is printed on an edge portion of the contact layer.

7. The apparatus of any one of the preceding claims, further comprising a wireless communications device electronically coupled with the electronic circuitry, the wireless communications device configured to transmit a signal containing at least one of an angle or a range of movement of the joint responsive to changes in the resistance of the at least one strain sensor.

8. The apparatus of any one of the preceding claims, further comprising a source of power for at least the electronic circuitry, wherein the source of power comprises one or more flexible batteries.

9. The apparatus of any one of the preceding claims, wherein the at least one strain sensor comprises a first strain sensor and a second strain sensor spaced apart from the first strain sensor.

10. The apparatus of any one of the preceding claims, wherein the at least one strain sensor comprises a first strain sensor and a second strain sensor, wherein the second strain sensor is parallel to the first strain sensor.

11. The apparatus of any one of the preceding claims, wherein the electronic circuitry is further configured to automatically collect data from the strain sensor at a predetermined frequency.

12. The apparatus of any one of the preceding claims, wherein the electronic circuitry is further configured to: automatically gather readings from the strain sensor at a plurality of time intervals and/or shorten a time interval in response to the strain sensor experiencing changes in the resistance at a higher frequency.

13. The apparatus of any one of the previous claims, further comprising an alarm, and wherein the electronic circuitry is further configured to activate the alarm in response to a resistance of the strain sensor satisfying a threshold resistance.

14. The apparatus of any one of the previous claims, wherein the contact layer is configured to have a shape which substantially matches a joint when the contact layer is in a relaxed state, wherein the joint is an elbow joint, a wrist joint, a knee joint, a neck joint, a spine, a finger joint, or an ankle joint.

15. The apparatus of any one of the previous claims, further comprising a canister configured to collect fluid that is aspirated from a wound under the contact layer.

**Patentansprüche**

1. Überwachungs- und/oder Therapieeinrichtung (200, 300, 320, 320'), umfassend:

    eine Kontaktschicht (202, 302, 322, 322'), die ein dehnbares oder im Wesentlichen dehnbares Substrat umfasst, das dazu ausgelegt ist, an einem Gelenk eines Benutzers angebracht zu werden;
    mindestens einen Dehnungssensor (500, 520, 540), der mit der Kontaktschicht (202, 302, 322, 322') gekoppelt ist;
    eine elektronische Schaltung (210), die elektronisch mit dem mindestens einen Dehnungssensor gekoppelt ist, wobei die elektronische Schaltung (210) dazu ausgelegt ist,
    einen Betrag einer Streckung des mindestens einen Dehnungssensors basierend auf einer Änderung eines Widerstands des mindestens einen Dehnungssensors (500, 520, 540) zu bestimmen; und
    eine Unterdruckquelle, die dazu ausgelegt ist, in Fluidverbindung mit einem Raum unter der Kontaktschicht (202, 302, 322, 322') zu stehen und Unterdruck an den Raum zu liefern;
    wobei:

    der mindestens eine Dehnungssensor (500, 520, 540) mehrere dehnbare Bahnen (204, 304, 324, 324') umfasst, die auf dem Substrat positioniert sind;

sich der Widerstand des mindestens einen Dehnungssensors erhöht, wenn zumindest einige Bahnen der mehreren dehnbaren Bahnen aufgrund dessen, dass die Kontaktschicht gestreckt wird, gestreckt werden;

die elektronische Schaltung dazu ausgelegt ist, einen Winkel und/oder einen Bewegungsbereich des Gelenks als Reaktion auf Änderungen des Widerstands des mindestens einen Dehnungssensors (500, 520, 540) zu bestimmen;

wobei die elektronische Schaltung einen maschinenlesbaren Speicher umfasst, der dazu ausgelegt ist, Informationen in Bezug auf eine Streckung des mindestens einen Dehnungssensors aufzuzeichnen;

die elektronische Schaltung dazu ausgelegt ist, automatisch Daten von dem Dehnungssensor mit einer vorbestimmten Frequenz zu sammeln; und

die Einrichtung ferner einen Temperatursensor umfasst, wobei der Temperatursensor dazu ausgelegt ist, der elektronischen Schaltung ein elektrisches Signal in Bezug auf eine Temperatur des Dehnungssensors und/oder eine Temperatur eines Gebiets des Substrats angrenzend an den Dehnungssensor bereitzustellen.

2.  Einrichtung nach Anspruch 1, wobei die mehreren dehnbaren Bahnen leitfähige Tinte umfassen, die auf das Substrat gedruckt ist.

3.  Einrichtung nach Anspruch 1 oder Anspruch 2, wobei die elektronische Schaltung mit der Kontaktschicht gekoppelt oder von der Kontaktschicht beabstandet ist.

4.  Einrichtung nach einem der vorhergehenden Ansprüche, wobei zumindest ein Teil der elektronischen Schaltung direkt auf die Kontaktschicht gedruckt ist.

5.  Einrichtung nach einem der vorhergehenden Ansprüche, wobei die elektronische Schaltung zumindest teilweise innerhalb eines Gehäuses (310, 328) positioniert ist, das dazu ausgelegt ist, in einem Abstand von der Kontaktschicht positionierbar zu sein, so dass die elektronische Schaltung von der Kontaktschicht beabstandet ist, und wobei das Gehäuse (310, 328) mit der Kontaktschicht gekoppelt sein kann.

6.  Einrichtung nach einem der vorhergehenden Ansprüche, wobei der mindestens eine Dehnungssensor und/oder die elektronische Schaltung auf einem Randabschnitt der Kontaktschicht positioniert oder auf diesen gedruckt ist.

7.  Einrichtung nach einem der vorhergehenden Ansprüche, ferner umfassend eine drahtlose Kommunikationsvorrichtung, die elektronisch mit der elektronischen Schaltung gekoppelt ist, wobei die drahtlose Kommunikationsvorrichtung dazu ausgelegt ist, ein Signal, das mindestens eines eines Winkels oder eines Bewegungsbereichs des Gelenks enthält, als Reaktion auf Änderungen des Widerstands des mindestens einen Dehnungssensors zu übertragen.

8.  Einrichtung nach einem der vorhergehenden Ansprüche, ferner umfassend eine Leistungsquelle für mindestens die elektronische Schaltung, wobei die Leistungsquelle eine oder mehrere flexible Batterien umfasst.

9.  Einrichtung nach einem der vorhergehenden Ansprüche, wobei der mindestens eine Dehnungssensor einen ersten Dehnungssensor und einen von dem ersten Dehnungssensor beabstandeten zweiten Dehnungssensor umfasst.

10. Einrichtung nach einem der vorhergehenden Ansprüche, wobei der mindestens eine Dehnungssensor einen ersten Dehnungssensor und einen zweiten Dehnungssensor umfasst, wobei der zweite Dehnungssensor parallel zu dem ersten Dehnungssensor ist.

11. Einrichtung nach einem der vorhergehenden Ansprüche, wobei die elektronische Schaltung ferner dazu ausgelegt ist, automatisch Daten von dem Dehnungssensor mit einer vorbestimmten Frequenz zu sammeln.

12. Einrichtung nach einem der vorhergehenden Ansprüche, wobei die elektronische Schaltung ferner zu Folgendem ausgelegt ist: automatisches Sammeln von Messwerten von dem Dehnungssensor in mehreren Zeitintervallen und/oder Verkürzen eines Zeitintervalls als Reaktion darauf, dass der Dehnungssensor Änderungen des Widerstands mit einer höheren Frequenz erfährt.

13. Einrichtung nach einem der vorhergehenden Ansprüche, ferner umfassend einen Alarm, und wobei die elektronische Schaltung ferner dazu ausgelegt ist, den Alarm als Reaktion darauf zu aktivieren, dass ein Widerstand des Dehnungssensors einen Schwellenwiderstand erfüllt.

14. Einrichtung nach einem der vorhergehenden Ansprüche, wobei die Kontaktschicht dazu ausgelegt ist, eine Form aufzuweisen, die im Wesentlichen einem Gelenk entspricht, wenn sich die Kontaktschicht in einem entspannten Zustand befindet, wobei das Gelenk ein Ellbogengelenk, ein Handgelenk, ein Kniegelenk, ein Halsgelenk, eine Wirbelsäule, ein Fingergelenk oder ein Knöchelgelenk ist.

15. Einrichtung nach einem der vorhergehenden Ansprüche, ferner umfassend einen Behälter, der dazu ausgelegt ist, Fluid zu sammeln, das aus einer Wunde unter der Kontaktschicht abgesaugt wird.

**Revendications**

1. Appareil de surveillance et/ou de thérapie (200, 300, 320, 320'), comprenant :

   une couche de contact (202, 302, 322, 322') comprenant un substrat étirable ou sensiblement étirable configuré pour être fixé à une articulation d'un utilisateur ;
   au moins un capteur de contrainte (500, 520, 540) couplé à la couche de contact (202, 302, 322, 322') ;
   une circuiterie électronique (210) couplée électroniquement à l'au moins un capteur de contrainte, la circuiterie électronique (210) étant configurée pour déterminer une quantité d'un allongement de l'au moins un capteur de contrainte sur la base d'une variation de résistance de l'au moins un capteur de contrainte (500, 520, 540) ; et
   une source de pression négative configurée pour être en communication fluidique avec un espace situé sous la couche de contact (202, 302, 322, 322') et pour fournir une pression négative à l'espace ;
   et dans lequel :

   l'au moins un capteur de contrainte (500, 520, 540) comprend une pluralité de pistes étirables (204, 304, 324, 324') positionnées sur le substrat ;
   la résistance de l'au moins un capteur de contrainte augmente à mesure qu'au moins certaines pistes parmi la pluralité de pistes étirables sont allongées en raison de l'allongement de la couche de contact ;
   la circuiterie électronique est configurée pour déterminer un angle et/ou une amplitude de mouvement de l'articulation en réponse à des variations de la résistance de l'au moins un capteur de contrainte (500, 520, 540) ;
   la circuiterie électronique comprenant une mémoire lisible par machine, configurée pour enregistrer des informations relatives à un allongement de l'au moins un capteur de contrainte ;
   la circuiterie électronique étant configurée pour collecter automatiquement des données du capteur de contrainte à une fréquence prédéterminée ; et
l'appareil comprenant en outre un capteur de température, le capteur de température étant configuré pour fournir à la circuiterie électronique un signal électrique lié à une température du capteur de contrainte et/ou à une température d'une région du substrat adjacente au capteur de contrainte.

2. Appareil selon la revendication 1, la pluralité de pistes étirables comprenant une encre conductrice imprimée sur le substrat.

3. Appareil selon la revendication 1 ou 2, la circuiterie électronique étant couplée à la couche de contact ou espacée de la couche de contact.

4. Appareil selon l'une quelconque des revendications précédentes, au moins une partie de la circuiterie électronique étant imprimée directement sur la couche de contact.

5. Appareil selon l'une quelconque des revendications précédentes, la circuiterie électronique étant positionnée au moins en partie à l'intérieur d'un boîtier (310, 328) qui est configuré pour pouvoir être positionné à une distance de la couche de contact de telle sorte que la circuiterie électronique soit espacée de la couche de contact, et le boîtier (310, 328) pouvant être couplé à la couche de contact.

6. Appareil selon l'une quelconque des revendications précédentes, l'au moins un capteur de contrainte et/ou la circuiterie électronique étant positionnés sur une partie périphérique de la couche de contact ou étant imprimés sur celle-ci.

7. Appareil selon l'une quelconque des revendications précédentes, comprenant en outre un dispositif de communication sans fil couplé électroniquement à la circuiterie électronique, le dispositif de communication sans fil étant configuré pour émettre un signal contenant un angle et/ou une amplitude de mouvement de l'articulation en réponse à des variations de la résistance de l'au moins un capteur de contrainte.

8. Appareil selon l'une quelconque des revendications précédentes, comprenant en outre une source d'alimentation pour au moins la circuiterie électronique, la source d'alimentation comprenant une ou plusieurs batteries flexibles.

9. Appareil selon l'une quelconque des revendications précédentes, l'au moins un capteur de contrainte comprenant un premier capteur de contrainte et un second capteur de contrainte espacé du premier capteur de contrainte.

10. Appareil selon l'une quelconque des revendications précédentes, l'au moins un capteur de contrainte comprenant un premier capteur de contrainte et un second capteur de contrainte, le second capteur de contrainte étant parallèle au premier capteur de contrainte.

11. Appareil selon l'une quelconque des revendications précédentes, la circuiterie électronique étant en outre configurée pour collecter automatiquement des données du capteur de contrainte à une fréquence prédéterminée.

12. Appareil selon l'une quelconque des revendications précédentes, la circuiterie électronique étant en outre configurée pour : recueillir automatiquement des relevés du capteur de contrainte à une pluralité d'intervalles de temps et/ou raccourcir un intervalle de temps en réponse au fait que le capteur de contrainte subit des variations de la résistance à une fréquence plus élevée.

13. Appareil selon l'une quelconque des revendications précédentes, comprenant en outre une alarme, et la circuiterie électronique étant en outre configurée pour activer l'alarme en réponse au fait qu'une résistance du capteur de contrainte satisfait à un seuil de résistance.

14. Appareil selon l'une quelconque des revendications précédentes, la couche de contact étant configurée pour avoir une forme qui correspond sensiblement à une articulation lorsque la couche de contact est dans un état détendu, l'articulation étant une articulation du coude, une articulation du poignet, une articulation du genou, une articulation du cou, une colonne vertébrale, une articulation du doigt ou une articulation de la cheville.

15. Appareil selon l'une quelconque des revendications précédentes, comprenant en outre une boîte configurée pour collecter un fluide qui est aspiré d'une plaie sous la couche de contact.

KNEEPAD

FIGURE 1B

ANKLET

FIGURE 1C

FIGURE 1A

FIGURE 1D

```
┌─────────────────┐              ┌──────────────────────┐
│  STRAIN SENSOR  │──────────────│ ELECTRONIC CIRCUITRY │
│       420       │              │         430          │
└─────────────────┘              └──────────────────────┘
┌────────────────────────────────────────────────────────┐
│                          410                           │
└────────────────────────────────────────────────────────┘
```

# FIGURE 2

# FIGURE 3

204

210

**FIGURE 4**

300

304

310

312

314

302

**FIGURE 5**

**FIGURE 6**

**FIGURE 7**

**FIGURE 8**

500

512

502

504

508

510

**FIGURE 9**

520

502

504

512

502

522 524 522 524 521 524 522

**FIGURE 10**

540

512

546

510

542

544

**FIGURE 11**

540

542

544

542

DS

544

540

542

544

C    R1    DS
         R2

**FIGURE 12**

R1

R2

A

**FIGURE 13**

FIGURE 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1918475 A
- GB 1918680 A
- WO 2019096828 A
- WO 2019042790 A
- WO 10061225 A
- US 2016114074 A
- US 20060142560 A
- US 5703225 A
- WO 2013007973 A
- GB 1618298 A
- GB 1621057 A
- GB 1709987 A
- EP 2498829 A
- EP 1718257 A
- WO 9958090 A
- WO 2006110527 A
- US 6759566 B
- US 20020099318 A
- EP 2019065602 W

### Non-patent literature cited in the description

- Highly-Stretchable Biomechanical Strain Sensor using Printed Liquid Metal Paste. **VOTZKE CALLEN et al.** 2018 IEEE BIOMEDICAL CIRCUITS AND SYSTEMS CONFERENCE (BIOCAS). IEEE, 17 October 2018, 1-4